(19) Europäisches Patentamt
European Patent Office
Office européen des brevets

(11) EP 3 075 400 A1

# (12) EUROPEAN PATENT APPLICATION
published in accordance with Art. 153(4) EPC

(43) Date of publication:
**05.10.2016 Bulletin 2016/40**

(21) Application number: **14865433.8**

(22) Date of filing: **27.11.2014**

(51) Int Cl.:
***A61L 33/00*** *(2006.01)*

(86) International application number:
**PCT/JP2014/081306**

(87) International publication number:
**WO 2015/080176 (04.06.2015 Gazette 2015/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.11.2013 JP 2013246581**
**12.02.2014 JP 2014024626**

(71) Applicant: **Toray Industries, Inc.**
**Tokyo 103-8666 (JP)**

(72) Inventors:
- **FUJITA, Masaki**
**Otsu-shi**
**Shiga 520-8558 (JP)**
- **KADOWAKI, Koji**
**Otsu-shi**
**Shiga 520-8558 (JP)**
- **SAKAGUCHI, Yuka**
**Otsu-shi**
**Shiga 520-8558 (JP)**
- **TANAHASHI, Kazuhiro**
**Otsu-shi**
**Shiga 520-8558 (JP)**

(74) Representative: **Pillep, Bernhard**
**Kador & Partner**
**Corneliusstrasse 15**
**80469 München (DE)**

## (54) ANTI-THROMBOTIC MATERIAL

(57) The present invention aims to provide an antithrombogenic material in which an antithrombogenic compound other than heparin or heparin derivatives is immobilized on a base material to prevent elution of the compound having antithrombogenicity from the base material, which antithrombogenic material therefore maintains high antithrombogenicity for a long period. The present invention provides an antithrombogenic material including: a coating material containing a skeletal structure composed of a polymer, a skeletal structure composed of 4-(aminomethyl)benzenecarboxyimidamide or benzene amidine, and a skeletal structure composed of methoxybenzenesulfonic acid amide; and a base material whose surface is coated with the coating material; wherein the coating material is covalently bound to the base material, and the peak abundance ratio of the carbonyl-derived component in the total component of the Cls peak as measured by X-ray photoelectron spectroscopy on the surface is not less than 1.0 atomic percent.

EP 3 075 400 A1

## Description

TECHNICAL FIELD

The present invention relates to an antithrombogenic material.

BACKGROUND ART

Medical equipments and medical instruments (artificial kidneys, artificial lungs, artificial blood vessels, artificial valves, stents, stent-grafts, catheters, free-thrombus capture devices, angioscopes, sutures, blood circuits, tubes, cannulae, blood bags, syringes, and the like) need to be composed of antithrombogenic materials having high antithrombogenicity so that blood coagulation can be avoided on the surface in contact with blood. Conventionally, in order to increase antithrombogenicity of the base material to be used for a medical equipment or medical instrument, methods in which heparin or a heparin derivative, which is an inexpensive and time-proven anticoagulant, is attached to a surface of the base material has been employed.

Examples of the method for attaching heparin or a heparin derivative to a surface of a base material to be used for an antithrombogenic material include methods in which the heparin or heparin derivative is immobilized by covalent bonding to a functional group introduced on the surface of the base material to be used for the antithrombogenic material (Patent Documents 1 to 3), and methods in which the heparin or heparin derivative is immobilized by ionic bonding to a positively charged cationic compound introduced on the surface of the base material to be used for the antithrombogenic material (Patent Documents 4 to 10).

Examples of known methods for attaching an antithrombogenic compound other than heparin or heparin derivatives to a surface of a base material to be used for an antithrombogenic material include methods in which a compound that inhibits a plurality of blood coagulation factors involved in blood coagulation reaction, in particular, a compound that inhibits both of platelets, which are involved in the stage of primary hemostasis, and thrombin, which is involved in the stage of thrombus formation, is attached to the surface of the base material.

Patent Document 11 discloses a method in which a solution of a compound having an action to inhibit blood coagulation factors, that is, having both anti-platelet adhesion capacity and antithrombin activation capacity, is brought into contact with a surface of a base material. Patent Document 12 discloses a example in which two kinds of compounds-a compound having anti-platelet adhesion capacity and a compound having antithrombin activation capacity-are immobilized by radiation, and used. Patent Document 13 discloses an example in which a compound having both anti-platelet adhesion capacity and antithrombin activation capacity is immobilized by radiation, and used.

Other examples of known methods for attaching an antithrombogenic compound other than heparin or heparin derivatives to a surface of a base material to be used for an antithrombogenic material include methods in which a compound that inhibits a plurality of blood coagulation factors involved in blood coagulation reaction, in particular, a compound that inhibits adhesion of platelets, which are involved in the stage of primary hemostasis, is attached to the surface of the base material. Examples of such a compound include betaine compounds (Patent Documents 14 to 16).

PRIOR ART DOCUMENTS

[Patent Documents]

[Patent Document 1] JP 4152075 B
[Patent Document 2] JP 3497612 B
[Patent Document 3] Japanese Translated PCT Patent Application Laid-open No. 10-513074
[Patent Document 4] JP 60-041947 B
[Patent Document 5] JP 60-047287 B
[Patent Document 6] JP 4273965 B
[Patent Document 7] JP 10-151192 A
[Patent Document 8] JP 3341503 B
[Patent Document 9] JP 3497612 B
[Patent Document 10] JP 3834602 B
[Patent Document 11] JP 4461217 B
[Patent Document 12] WO 08/032758
[Patent Document 13] WO 12/176861
[Patent Document 14] JP 54-63025 A

[Patent Document 15] JP 2000-279512 A
[Patent Document 16] JP 5349873 B

SUMMARY OF THE INVENTION

PROBLEMS TO BE SOLVED BY THE INVENTION

However, at present, methods in which heparin or a heparin derivative is attached to a surface of a base material, such as those in Patent Documents 1 to 10, cannot be used for patients with heparin-induced thrombocytopenia or patients with bleeding.
Heparin and heparin derivatives exhibit high antithrombogenicity when they are bound to antithrombin III (hereinafter referred to as "ATIII"). It is known, however, that their antithrombogenicity is insufficient in blood in which the ATIII level is low.
On the other hand, as a method for binding an antithrombogenic compound other than heparin or heparin derivatives to a surface of a base material, a method in which a solution of a compound having an action to inhibit blood coagulation factors, that is, having both anti-platelet adhesion capacity and antithrombin activation capacity, is brought into contact with a surface of a base material has been disclosed (Patent Document 11). However, since the antithrombogenic compound is attached to the surface of the base material by physical adsorption rather than covalent bonding, there is a concern that elution of the compound may occur, so that it is difficult for the compound to continuously exhibit high antithrombogenicity for a long period.
The compound having anti-platelet adhesion capacity and the compound having antithrombin activation capacity described in Patent Document 12, and the compound having both anti-platelet adhesion capacity and antithrombin activation capacity described in Patent Document 13, do not have a functional group that covalently binds to the base material by chemical reaction, so that those compounds need to be covalently bound to the surface of the base material by radiation imadiation. In such cases, as the radiation dose increases, the amount of radicals generated increases, leading to immobilization of a larger amount of the compound. However, it has been found that, in these cases, generation of radicals in nonspecific portions of the compound and their reaction with the base material cause structural changes of the compound, mainly leading to decreased antithrombin activation capacity. Although there are methods in which the radiation dose is decreased or a radical scavenger is added in order prevent the generation of radicals, an increase in the amount of the compound immobilized cannot be expected in these cases at present.
That is, an antithrombogenic material prepared using a coating material other than heparin or heparin derivatives, which antithrombogenic material can be easily immobilized on a surface of a base material and continuously exhibit high antithrombogenicity for a long period, has not been reported to date.
As methods for binding an antithrombogenic compound other than heparin or heparin derivatives to a surface of a base material, methods in which a betaine compound is attached to a surface of a base material has been disclosed (Patent Documents 14 to 16). Although betaine compounds are known to inhibit adhesion of platelets, those compounds cannot continuously exhibit high antithrombogenicity for a long period, and are not reported to have antithrombin activity.
That is, there is no case in which a betaine compound is immobilized on a surface of a base material to allow long-term maintenance of high antithrombogenicity, and there is no antithrombogenic material to which both an antithrombogenic compound other than heparin or heparin derivatives, and a betaine compound, are covalently bound at present.
Under such circumstances, an object of the present invention is to provide an antithrombogenic material in which an antithrombogenic compound other than heparin or heparin derivatives is immobilized on a base material such that elution of the antithrombogenic compound from the base material is prevented, which antithrombogenic material can therefore continuously exhibit high antithrombogenicity for a long period.
The present invention also aims to provide an antithrombogenic material in which both an antithrombogenic compound which is heparin or a heparin derivative and a betaine compound are immobilized on a base material such that elution of the antithrombogenic compounds from the base material is prevented, which antithrombogenic material can therefore continuously have high antithrombogenicity for a long period.

MEANS FOR SOLVING THE PROBLEMS

As a result of intensive study to solve the above problems, the present inventors discovered the inventions of (1) to (11) described below.

(1) An antithrombogenic material comprising:

a coating material containing: a skeletal structure composed of a polymer containing, as a constituent monomer,

a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; a skeletal structure composed of 4-(aminomethyl)benzenecarboxyimidamide or benzene amidine; and a skeletal structure composed of methoxybenzenesulfonic acid amide; and

a base material whose surface is coated with the coating material;

wherein

the coating material is covalently bound to the base material; and

the peak abundance ratio of the carbonyl-derived component in the total component of the C1s peak as measured by X-ray photoelectron spectroscopy (XPS) on the surface is not less than 1.0 atomic percent.

(2) The antithrombogenic material according to (1), wherein the skeletal structure composed a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; the skeletal structure composed of 4-(aminomethyl)benzenecarboxyimidamide or benzene amidine; and the skeletal structure composed of methoxybenzenesulfonic acid amide; are the compound represented by the following General Formula (I):

[Chemical Formula 1]

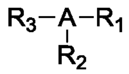

(I)

[wherein $R_1$ represents the polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; $R_2$ represents 4-(aminomethyl)benzenecarboxyimidamide or benzene amidine; $R_3$ represents methoxybenzenesulfonic acid amide; and A represents an arbitrary covalent bond(s)].

(3) The antithrombogenic material according to (1) or (2), wherein the coating material comprises at least one of a betaine compound, cationic polymer, anionic polymer, and anionic compound, and has an antithrombogenic action.

(4) The antithrombogenic material according to (2) or (3), wherein

the coating material comprises a betaine compound;

each of the compound represented by General Formula (I) and the betaine compound is covalently bound to the base material; and

the coating material has an antithrombogenic action.

(5) The antithrombogenic material according to (2) or (3), wherein

the coating material comprises at least one of a cationic polymer, anionic polymer, and anionic compound;

each of the compound represented by General Formula (I) and at least one of the cationic polymer, anionic polymer, and anionic compound is covalently bound to the base material; and

the coating material has an antithrombogenic action.

(6) The antithrombogenic material according to (2) or (3), wherein

the coating material comprises at least one of a cationic polymer, anionic polymer, and anionic compound;

at least one of the cationic polymer, anionic polymer, and anionic compound is covalently bound to the base material;

the compound represented by General Formula (I) is covalently bound to any one of the cationic polymer, anionic polymer, and anionic compound; and

the coating material has an antithrombogenic action.

(7) The antithrombogenic material according to (2) or (3), wherein

the coating material comprises a betaine compound, and at least one of a cationic polymer, anionic polymer, and anionic compound;

at least one of the cationic polymer, anionic polymer, and anionic compound is covalently bound to the base material;

each of the compound represented by General Formula (I) and the betaine compound is covalently bound to any one of the cationic polymer, anionic polymer, and anionic compound; and

the coating material has an antithrombogenic action.

(8) The antithrombogenic material according to any of (2) to (7), wherein the compound represented by General Formula (I) is a compound represented by any of the following General Formulae (II) to (V):

[Chemical Formula 2]

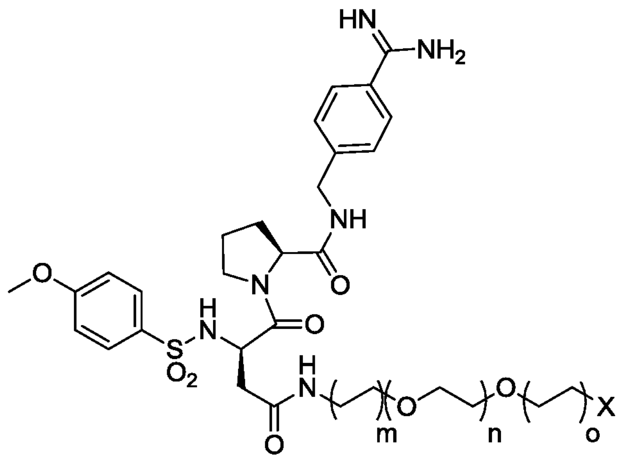

(II)

[Chemical Formula 3]

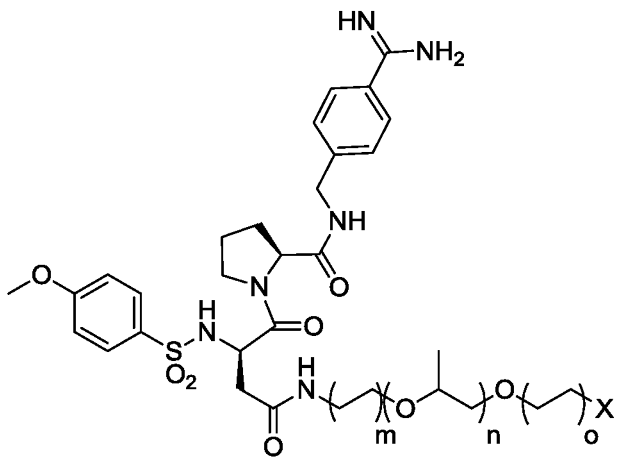

(III)

[Chemical Formula 4]

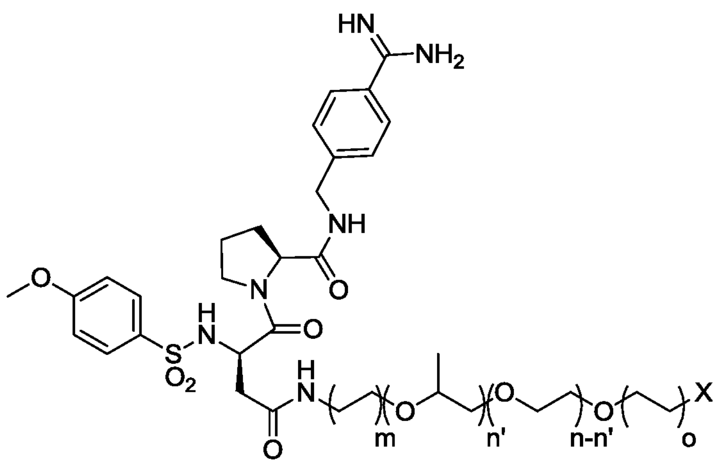

(IV)

[Chemical Formula 5]

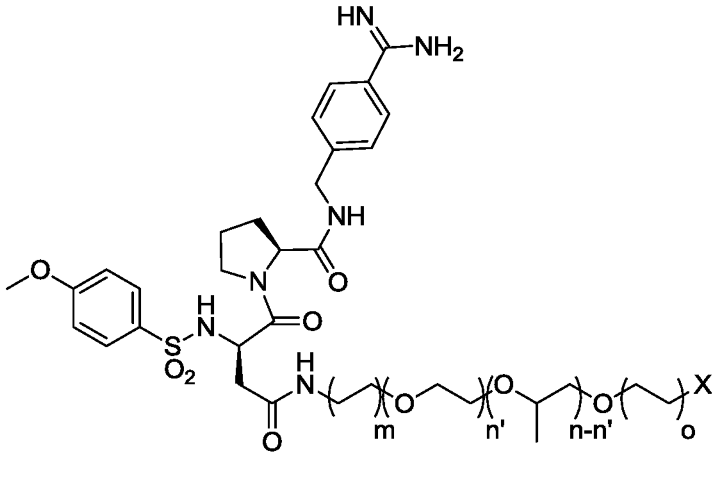

(V)

[wherein each of m and o represents an integer of 0 to 4; n represents an integer of 3 to 1000, and n' represents an integer of 3 to 1000, with the proviso that n≥n'; and X represents a functional group selected from the group consisting of hydroxyl, thiol, amino, carboxyl, aldehyde, isocyanate, and thioisocyanate].

(9) The antithrombogenic material according to any of (2) to (8), wherein

the cationic polymer is a polymer comprising, as a constituent monomer, a compound selected from the group consisting of alkyleneimines, vinylamines, allylamines, lysine, protamine, and diallyldimethylammonium chloride;

the anionic polymer is a polymer comprising, as a constituent monomer, a compound selected from the group consisting of acrylic acid, methacrylic acid, α-glutamic acid, γ-glutamic acid, and aspartic acid; and

the anionic compound is a compound selected from the group consisting of oxalic acid, malonic acid, succinic acid, fumaric acid, glutaric acid, adipic acid, pimelic acid, suberic acid, azelaic acid, sebacic acid, malic acid, tartaric acid, and citric acid.

(10) The antithrombogenic material according to any of (1) to (9), wherein the coating material has a thickness of 1 to 600 nm and is present on the surface of the base material.

(11) The antithrombogenic material according to any of (1) to (10), wherein the coating material is present to a depth of 15 to 200 nm from the interface of the base material.

As a result of intensive study to solve the above problems, the present inventors also discovered the inventions of (12) to (14) described below.

(12) An antithrombogenic material comprising:

a coating material containing: a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; a skeletal structure composed of benzene amidine; and a skeletal structure composed of methoxybenzenesulfonic acid amide; and

a base material whose surface is coated with the coating material;

wherein

the coating material is covalently bound to the base material; and

the peak abundance ratio of the carbonyl-derived component in the total component of the C1s peak as measured by X-ray photoelectron spectroscopy (XPS) on the surface is not less than 1.0 atomic percent.

(13) An antithrombogenic material in which an antithrombogenic compound represented by the General Formula (I) below:

[Chemical Formula 6]

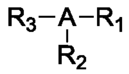
(I)

[wherein $R_1$ represents a hydrophilic polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; $R_2$ represents methoxybenzenesulfonic acid amide; $R_3$ represents 4-(aminomethyl)benzenecarboxyimidamide or benzene amidine; and A represents an arbitrary covalent bond(s)]
and betaine are covalently bound to a base material, and exhibit an antithrombogenic action.
(14) An antithrombogenic material comprising a cationic polymer and at least one of an anionic polymer and anionic compound,
wherein
the cationic polymer and either one of the anionic polymer and the anionic compound are covalently bound to a base material; and
an antithrombogenic compound represented by the General Formula (I) below:

[Chemical Formula 7]

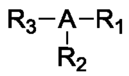
(I)

[wherein $R_1$ represents a hydrophilic polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; $R_2$ represents methoxybenzenesulfonic acid amide; $R_3$ represents 4-(aminomethyl)benzenecarboxyimidamide or benzene amidine; and A represents an arbitrary covalent bond(s)]
and betaine are covalently bound to either one of the cationic polymer and the anionic polymer or the anionic compound, and exhibit an antithrombogenic action.

## EFFECT OF THE INVENTION

In the antithrombogenic material of the present invention, an antithrombogenic compound can be easily immobilized on a surface of a base material, while elution of the compound from the base material can be suppressed, so that the antithrombogenic material can continuously exhibit high antithrombogenicity for a long period. The antithrombogenic material of the present invention can also be used for treatment of patients with heparin-induced thrombocytopenia, patients with bleeding, and patients in which the ATIII level is low, and can be favorably used for medical equipments and medical instruments (artificial kidneys, artificial lungs, artificial blood vessels, artificial valves, stents, stent-grafts, catheters, free-thrombus capture devices, angioscopes, sutures, blood circuits, tubes, cannulae, blood bags, syringes, and the like).

## MODE FOR CARRYING OUT THE INVENTION

The antithrombogenic material of the present invention is characterized in that it comprises:

a coating material containing: a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; a skeletal structure composed of 4-(aminomethyl)benzenecarboxyimidamide or benzene amidine; and a skeletal structure composed of methoxybenzenesulfonic acid amide; and
a base material whose surface is coated with the coating material;

wherein

the coating material is covalently bound to the base material; and
the peak abundance ratio of the carbonyl-derived component in the total component of the C1s peak as measured by X-ray photoelectron spectroscopy (XPS) on the surface is not less than 1.0 atomic percent.

The following terms used in the present description are defined as described below unless otherwise specified.

The term "antithrombogenicity" herein means a property which prevents blood coagulation on a surface in contact with blood. For example, "antithrombogenicity" means a property which inhibits platelet aggregation, or blood coagulation which proceeds due to, for example, activation of blood coagulation factors represented by thrombin.

The term "antithrombogenic material" herein means a material having antithrombogenicity. The "antithrombogenic material" may, but does not necessarily need to, be used as a material constituting medical equipments and medical instruments (artificial kidneys, artificial lungs, artificial blood vessels, artificial valves, stents, stent-grafts, catheters, free-thrombus capture devices, angioscopes, sutures, blood circuits, tubes, cannulae, blood bags, syringes, and the like). These medical equipments and medical instruments are frequently brought into contact with blood, and blood coagulation is likely to proceed on surfaces of the medical equipments and medical instruments. Therefore, antithrombogenic materials need to be used as materials for such medical equipments and medical instruments.

Among the materials constituting an antithrombogenic material, the "base material" means a substance constituting a surface to be coated with the coating material defined below. Preferred examples of the material of the base material in the present invention include, but are not limited to, polyester polymers, expanded porous polytetrafluoroethylene (hereinafter referred to as "ePTFE"), polyurethane, polyetherurethane, polyamide, vinyl chloride, polycarbonate, polystyrene, polyethylene, polypropylene, polymethylpentene, and polymethyl methacrylate. Among these, polyester polymers are preferred as the base material of the antithrombogenic material because of their versatility, and polymers containing at least an ester as a constituent monomer are more preferred. Examples of the polymers include polyethylene terephthalate (hereinafter referred to as "PET"), polytrimethylene terephthalate, polybutylene terephthalate, polyethylene naphthalate, and polybutylene naphthalate. Among these, PET is more preferred as the base material of the antithrombogenic material because of its versatility.

The "coating material" means a material with which at least a part of the surface of the base material is coated, and the coating material in the present invention contains three kinds of skeletal structures-a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; a skeletal structure composed of 4-(aminomethyl)benzenecarboxyimidamide or benzene amidine; and a skeletal structure composed of methoxybenzenesulfonic acid amide-in the coating material.

The three kinds of skeletal structures may be contained in separate compounds, or at least two skeletal structures may be bound to a compound by covalent bonding or ionic bonding. From the viewpoint of maintaining high antithrombogenicity for a long period, it is preferred to use, as the coating material, a compound containing all of these structures, that is, the skeletal structure composed of a polymer, the skeletal structure composed of 4-(aminomethyl)benzenecarboxyimidamide or benzene amidine, and the skeletal structure composed of methoxybenzenesulfonic acid amide.

The three kinds of skeletal structures preferably have a functional group(s) selected from the group consisting of, for example, hydroxyl, thiol, amino, carboxyl, aldehyde, isocyanate, and thioisocyanate, more preferably have amino or carboxyl, still more preferably have amino. The functional group(s) is/are preferably contained in the skeletal structure composed of a polymer. The functional group(s) is/are more preferably present at a terminus/termini of the skeletal structure composed of a polymer.

Using the functional group(s) selected from the group consisting of hydroxyl, thiol, amino, carboxyl, aldehyde, isocyanate, and thioisocyanate, the base material and the skeletal structures can be bound to each other, for example, through a disulfide bond(s), amide bond(s), ester bond(s), and/or urethane bond(s), and/or through a bond(s) formed by condensation reaction.

In the present invention, the present inventors intensively studied to increase especially anti-platelet adhesion capacity using an antithrombogenic compound other than heparin or heparin derivatives while elution of the compound is suppressed and high antithrombogenicity is maintained for a long period. As a result, the present inventors discovered that a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane is important therefor.

The skeletal structure composed of a polymer may be a copolymer with other monomers, or may be a modified body, as long as the effect of the present invention is not deteriorated. The modified body herein means a polymer which has the same monomer repeat units constituting the polymer, but has partially undergone, for example, radical decomposition or recombination due to the later-mentioned radiation imadiation.

The skeletal structure composed of a polymer is not limited as long as the constituent monomer described above is used, and may be either a homopolymer or a copolymer. In cases where the polymer is a copolymer, the copolymer may be any of a random copolymer, block copolymer, graft copolymer, and alternating copolymer. The skeletal structure composed of the polymer may be either linear or branched. More preferably, the skeletal structure composed

of the polymer has a hydrophilic functional group, and is a skeletal structure composed of a hydrophilic polymer soluble in water.

The homopolymer herein means a macromolecular compound obtained by polymerization of a single kind of constituent monomers. The copolymer herein means a macromolecular compound obtained by copolymerization of two or more kinds of monomers. The block copolymer means a copolymer having a molecular structure in which at least two kinds of polymers having different repeat units are covalently bound to each other to form a longer chain. The block means each of the "at least two kinds of polymers having different repeat units" constituting the block copolymer.

In the present invention, the present inventors intensively studied to increase especially antithrombin activation capacity using an antithrombogenic compound other than heparin or heparin derivatives while elution of the compound is suppressed and high antithrombogenicity is maintained for a long period. As a result, the present inventors discovered that a skeletal structure composed of 4-(aminomethyl)benzenecarboxyimidamide or benzene amidine, and a skeletal structure composed of methoxybenzenesulfonic acid amide, are important therefor.

The skeletal structure composed of 4-(aminomethyl)benzenecarboxyimidamide is any of the skeletal structures shown in the following General Formula (VI); the skeletal structure composed of benzene amidine is any of the skeletal structures shown in the following General Formula (VII); and the skeletal structure composed of methoxybenzenesulfonic acid amide is any of the skeletal structures shown in the following General Formula (VIII).

[Chemical Formula 8]

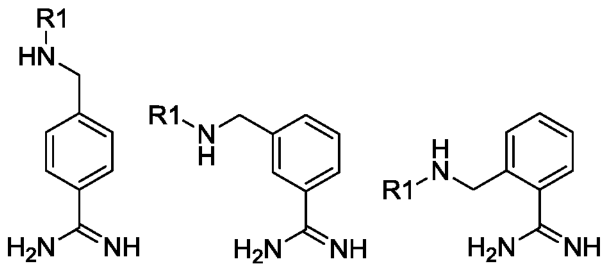
(VI)

[wherein R1 represents a moiety bound to another skeletal structure].

[Chemical Formula 9]

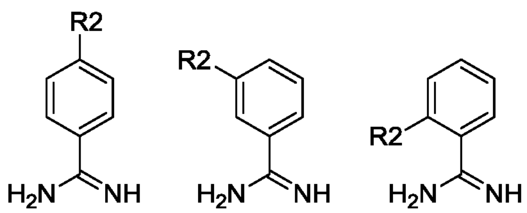
(VII)

[wherein R2 represents a moiety bound to another skeletal structure].

[Chemical Formula 10]

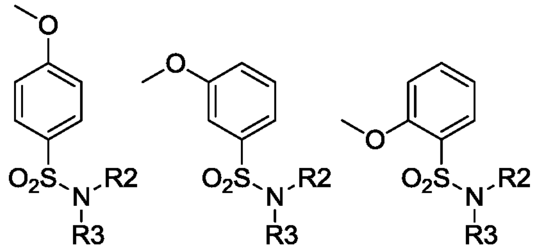
(VIII)

[wherein each of R3 and R4 represents a moiety bound to another skeletal structure].

In the present invention, as a compound containing all of the three kinds of structures used for the coating material, that is, the skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane, the skeletal structure composed of 4-(aminomethyl)benzenecarboxyimidamide or benzene amidine, and the skeletal structure composed of methoxybenzenesulfonic acid amide, a compound represented by the following General Formula (I) is preferred. In this General Formula, A is more preferably an amide bond.

[Chemical Formula 11]

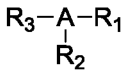

(I)

[wherein $R_1$ represents the polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; $R_2$ represents methoxybenzenesulfonic acid amide; $R_3$ represents 4-(aminomethyl)benzenecarboxyimidamide or benzene amidine; and A represents an arbitrary covalent bond(s)].

The compound represented by General Formula (I) as a compound containing all of the three kinds of structures used for the coating material, that is, the skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane, the skeletal structure composed of 4-(aminomethyl)benzenecarboxyimidamide or benzene amidine, and the skeletal structure composed of methoxybenzenesulfonic acid amide, is especially preferably a compound represented by any of the following General Formulae (II) to (V). In these General Formulae, X is preferably amino or carboxyl. X is more preferably amino.

[Chemical Formula 12]

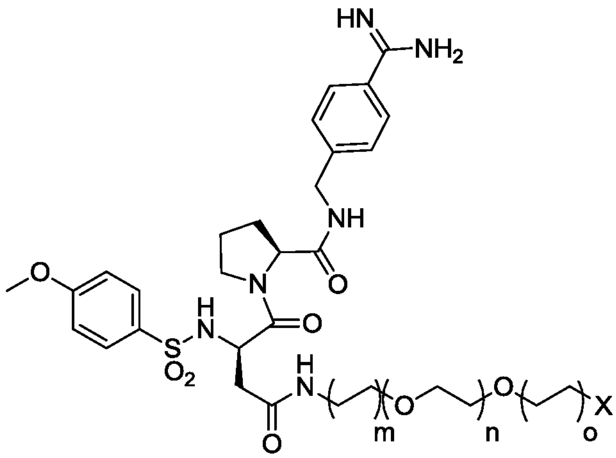

(II)

[Chemical Formula 13]

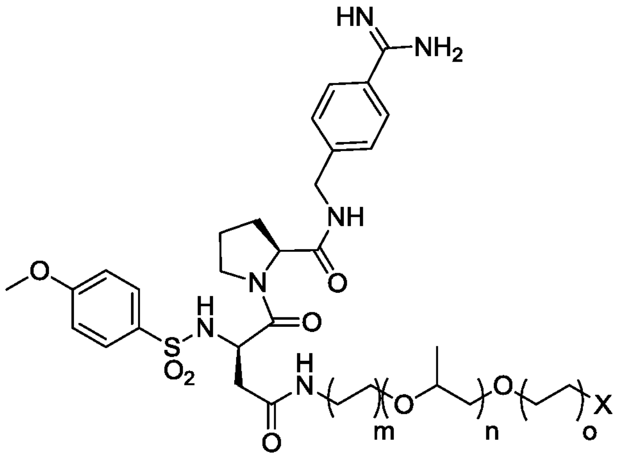
(III)

[Chemical Formula 14]

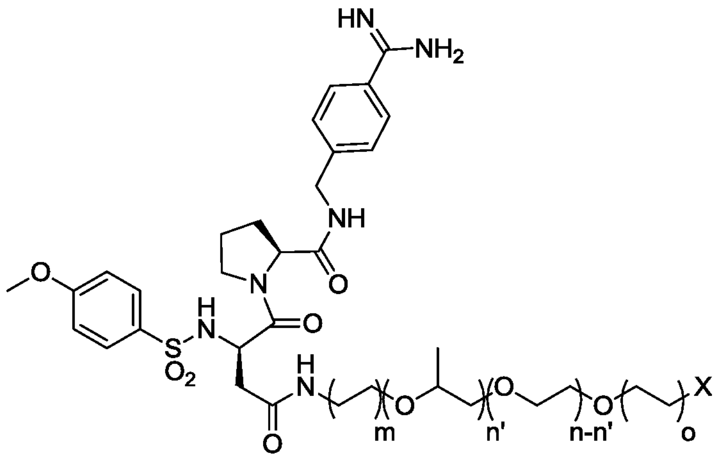
(IV)

[Chemical Formula 15]

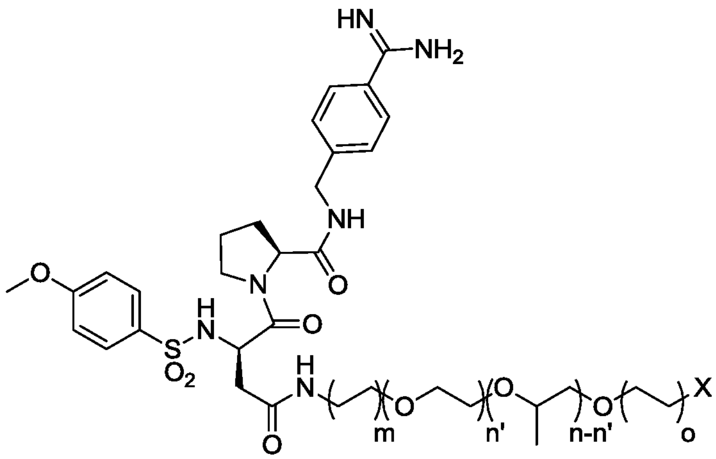
(V)

[wherein each of m and o represents an integer of 0 to 4; n represents an integer of 3 to 1000, and n' represents an integer of 3 to 1000, with the proviso that n≥n'; and X represents a functional group selected from the group consisting of hydroxyl, thiol, amino, carboxyl, aldehyde, isocyanate, and thioisocyanate].

In the present invention, it was discovered that, although X in the formulae described above may be contained in any of the skeletal structures, higher antithrombogenicity can be maintained for a long period in cases where the skeletal structure composed of a polymer, which has anti-platelet adhesion capacity, is present in the side in contact

with the base material, and the skeletal structure composed of 4-(aminomethyl)benzenecarboxyimidamide or benzene amidine and the skeletal structure composed of methoxybenzenesulfonic acid amide, which have antithrombin activation capacity, are present in the side in contact with blood, since the latter skeletal structures have higher thrombin capture capacity in such cases. That is, the reactive functional group (X in the formulae described above) to be covalently bound to the base material is more preferably contained in the skeletal structure composed of a polymer.

Using the reactive functional group X in the formulae, the base material and the coating material can be bound to each other, for example, through a disulfide bond(s), amide bond(s), ester bond(s), and/or urethane bond(s), and/or through a bond(s) formed by condensation reaction.

In the present invention, it was discovered that, in order to increase the coating amount of the coating material on the surface of the base material, and to maintain high antithrombogenicity for a longer period, the coating material preferably contains at least one of a betaine compound, cationic polymer, and anionic polymer or anionic compound, more preferably contains at least one of a betaine compound, cationic polymer, and an anionic polymer or anionic compound.

The betaine compound means a compound which has a positive charge and a negative charge at positions in the same molecule that are not adjacent to each other, wherein no dissociable hydrogen atom is bound to the atom having the positive charge, and the compound as a whole does not have a charge, or a salt thereof. In the present invention, the betaine compound is not limited as long as it is a compound containing a betaine compound in a part thereof. The betaine compound is preferably carboxybetaine, sulfobetaine, or phosphobetaine, more preferably carboxybetaine or sulfobetaine represented by the following General Formula (IX) or (X). X in General Formulae (IX) and (X) is preferably amino or carboxyl. X is more preferably amino.

[Chemical Formula 16]

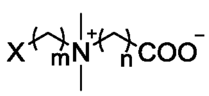

(IX)

[Chemical Formula 17]

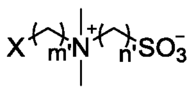

(X)

[wherein n represents any of 1 to 4; m represents an integer of 2 to 4; n' represents an integer of 2 to 4; m' represents an integer of 2 to 4; and X represents a functional group selected from the group consisting of hydroxyl, thiol, amino, carboxyl, aldehyde, isocyanate, and thioisocyanate].

From the viewpoint of maintenance of high antithrombogenicity for a long period, as well as from the viewpoint of safety, elution of the coating material into a body fluid such as blood is not preferred. Therefore, the coating material is preferably covalently bound to a surface of the base material. Since conventional antithrombogenic compounds other than heparin or heparin derivatives have no reactive functional group that covalently binds to a surface of a base material, those compounds are covalently bound by, for example, a method represented by radiation imadiation. However, absorption of high energy such as radiation causes structural changes due to radicals especially in the skeletal structure composed of 4-(aminomethyl)benzenecarboxyimidamide or benzene amidine and the skeletal structure composed of methoxybenzenesulfonic acid amide, which have antithrombin activation capacity, leading to a decrease in the antithrombin activation capacity. In contrast, in the present invention, a functional group(s) having reactivity with the base material is given to a compound(s) in the coating material, so that covalent bonds can be formed without using radiation imadiation.

From the viewpoint of maintenance of high antithrombogenicity for a long period, as well as from the viewpoint of safety, elution of a betaine compound into a body fluid such as blood is also not preferred. Therefore, the betaine compound is preferably covalently bound to a surface of the base material.

In the present invention, the present inventors intensively studied to increase especially anti-platelet adhesion capacity while elution of the compound is suppressed and high antithrombogenicity is maintained for a long period. As a result, the present inventors discovered that a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; and a betaine compound; are important therefor. It has been conventionally thought that betaine compounds do not largely

influence the antithrombin activation capacity. However, in the present invention, it was discovered that, in cases where both a skeletal structure composed of a polymer, and a betaine compound, are contained, both the anti-platelet adhesion capacity and the antithrombin activation capacity largely increase compared to cases where only one of the polymer and the betaine compound is bound to the base material.

From the viewpoint of maintenance of high antithrombogenicity for a long period, as well as from the viewpoint of safety, elution of a cationic polymer, and anionic polymer or anionic compound, into a body fluid such as blood is also not preferred. Therefore, the cationic polymer, and anionic polymer or anionic compound, are preferably covalently bound to a surface of the base material.

The term "covalent bond" herein means a chemical bond formed by sharing of an electron(s) between atoms. In the present invention, the covalent bond is a covalent bond between atoms such as carbon, nitrogen, oxygen, and/or sulfur present on the surfaces of the coating material and the base material, and may be a single bond or multiple bond. Examples of the type of the covalent bond include, but are not limited to, an amine bond, azide bond, amide bond, and imine bond. Among these, from the viewpoint of ease of formation of the covalent bond, stability after bonding, and the like, an amide bond is more preferred. As a result of intensive study, the present inventors discovered that formation of amide bonds between the surfaces of the coating material and the base material allows optimal configuration of the coating material on the surface of the base material, enabling maintenance of high antithrombogenicity for a longer period compared to cases where the coating material is covalently bound by radiation irradiation, even if the amount of the amide bonds introduced is small. Conformation of the covalent bonds is possible by judgment of whether elution occurs by washing with a solvent that dissolves the polymer.

Although appropriate orders of covalent bonding in the coating material are not limited in the present invention, modes of covalent bonding that are more preferred for allowing maintenance of high antithrombogenicity for a longer period are described below.

In the present invention, in order to increase the coating amount of the coating material on the surface of the base material, and to maintain high antithrombogenicity for a longer period, in cases where the coating material contains a betaine compound, the betaine compound is preferably covalently bound to the surface of the base material, and the compound represented by General Formula (I) is preferably covalently bound to the betaine compound. In such cases, the betaine compound is covalently bound to the base material as a compound that mediates binding of the compound represented by General Formula (I) to the base material. A part of the compound represented by General Formula (I) may also be covalently bound to the surface of the base material.

In the present invention, in order to increase the coating amount of the coating material on the surface of the base material, and to maintain high antithrombogenicity for a longer period, in cases where the coating material contains a cationic polymer and at least one of an anionic polymer and anionic compound, the cationic polymer and either one of the anionic polymer and anionic compound are preferably covalently bound to the surface of the base material, and the compound represented by General Formula (I) is preferably covalently bound to the cationic polymer and either one of the anionic polymer and anionic compound. In such cases, the cationic polymer and either one of the anionic polymer and anionic compound are bound to the base material as compounds that mediate binding of the compound represented by General Formula (I) to the base material. A part of the compound represented by General Formula (I) may also be covalently bound to the surface of the base material.

In cases where the coating material contains a betaine compound, cationic polymer, and at least one of an anionic polymer and anionic compound, the cationic polymer and either one of the anionic polymer and anionic compound are preferably covalently bound to the surface of the base material, and the compound represented by General Formula (I) and the betaine compound are preferably covalently bound to the cationic polymer and either one of the anionic polymer and anionic compound. In such cases, the cationic polymer and either one of the anionic polymer and anionic compound are bound to the base material as compounds that mediate binding of the compound represented by General Formula (I) and the betaine compound to the base material. A part of the compound represented by General Formula (I) and the betaine compound may also be covalently bound to the surface of the base material.

In the present invention, from the viewpoint of maintaining high antithrombogenicity for a longer period, the cationic polymer is preferably a polymer comprising, as a constituent monomer, a compound selected from the group consisting of alkyleneimines, vinylamines, allylamines, lysine, protamine, and diallyldimethylammonium chloride; the anionic polymer is preferably a polymer comprising, as a constituent monomer, a compound selected from the group consisting of acrylic acid, methacrylic acid, $\alpha$-glutamic acid, $\gamma$-glutamic acid, and aspartic acid; and the anionic compound is preferably a compound selected from the group consisting of dicarboxylic acids such as oxalic acid, malonic acid, succinic acid, fumaric acid, glutaric acid, adipic acid, pimelic acid, suberic acid, azelaic acid, sebacic acid, malic acid, tartaric acid, and dodecanedioic acid; and citric acid.

In particular, from the viewpoint of increasing the coating amount of the coating material on the surface of the base material, and maintaining high antithrombogenicity for a longer period, the coating material preferably contains a cationic polymer and at least one of an anionic polymer and anionic compound, more preferably contains both a cationic polymer, and an anionic polymer or anionic compound.

The cationic polymer is preferably, but does not necessarily need to be, a polyalkyleneimine since, in cases where the polymer is branched, a larger coating amount can be achieved by covalent bonding with the base material and the coating material. Examples of the polyalkyleneimine include polyethyleneimine (hereinafter referred to as "PEI"), polypropyleneimine, and polybutyleneimine, and alkoxylated polyalkyleneimine. PEI is more preferred.

Specific examples of the PEI include "LUPASOL" (registered trademark) (manufactured by BASF), and "EPOMIN" (registered trademark) (manufactured by Nippon Shokubai Co., Ltd.). The PEI may be a copolymer with one or more other monomers or a modified body as long as the effect of the present invention is not deteriorated.

The cationic polymer may, but does not necessarily need to, form a copolymer with one or more constituent monomers other than those described above. Examples of such monomers include ethylene glycol, propylene glycol, vinylpyrrolidone, vinyl alcohol, vinylcaprolactam, vinyl acetate, styrene, methyl methacrylate, hydroxyethyl methacrylate, and siloxane. The content of the constituent monomer(s) forming the copolymer with the cationic polymer is preferably not more than 10% by weight since the amount of coating formed by covalent bonding with the base material and the coating material is small in cases where the content is too large.

The anionic polymer is preferably, but does not necessarily need to be, a polyacrylic acid (hereinafter referred to as "PAA"), polymethacrylic acid, poly($\alpha$-glutamic acid), poly($\gamma$-glutamic acid), or polyaspartic acid since, in cases where the weight ratio of anionic functional groups is high, a larger coating amount can be achieved by covalent bonding with the base material and the coating material. The anionic polymer is more preferably PAA.

Specific examples of the PAA include "polyacrylic acid" (manufactured by Wako Pure Chemical Industries, Ltd.). The PAA may be a copolymer with one or more other monomers or a modified body as long as the effect of the present invention is not deteriorated.

The anionic polymer may, but does not necessarily need to, form a copolymer with one or more constituent monomers other than those described above. Examples of such monomers include ethylene glycol, propylene glycol, vinylpyrrolidone, vinyl alcohol, vinylcaprolactam, vinyl acetate, styrene, methyl methacrylate, hydroxyethyl methacrylate, and siloxane. The content of the constituent monomer(s) forming the copolymer with the anionic polymer is preferably not more than 10% by weight since the amount of coating formed by covalent bonding with the base material and the coating material is small in cases where the content is too large.

The anionic compound is preferably, but does not necessarily need to be, oxalic acid, malonic acid, succinic acid, fumaric acid, glutaric acid, adipic acid, pimelic acid, suberic acid, azelaic acid, sebacic acid, malic acid, tartaric acid, or citric acid since, in cases where the weight ratio of anionic functional groups is high, a larger coating amount can be achieved by covalent bonding with the base material and the coating material. Succinic acid is more preferred.

In the present invention, in cases where the number average molecular weight of the coating material containing: a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; a skeletal structure composed of 4-(aminomethyl)benzenecarboxyimidamide or benzene amidine; and a skeletal structure composed of methoxybenzenesulfonic acid amide; is too low, the anti-platelet adhesion capacity is low, so that the antithrombogenicity of interest is less likely to be obtained. On the other hand, in cases where the number average molecular weight of the coating material is too high, the anti-platelet adhesion capacity is high, but the antithrombogenicity of interest is less likely to be obtained since the portion which exhibits the antithrombin activation capacity is included in the inside. Therefore, the number average molecular weight of the coating material is preferably 1500 to 20,000, more preferably 2000 to 10,000. The number average molecular weight and the weight average molecular weight of the polymer can be measured by, for example, gel permeation chromatography or the light scattering method.

Similarly, in cases where the weight average molecular weight of the cationic polymer and the anionic polymer is too low, the amount of coating formed by covalent bonding with the base material and the coating material is small, so that high antithrombogenicity is less likely to be obtained. On the other hand, in cases where the weight average molecular weight of the cationic polymer and the anionic polymer is too high, the coating material is included in the inside. Therefore, the weight average molecular weight of the cationic polymer and the anionic polymer is preferably 600 to 2,000,000, more preferably 10,000 to 1,000,000.

More specifically, the presence of the skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane, the skeletal structure composed of 4-(aminomethyl)benzenecarboxyimidamide or benzene amidine, and the skeletal structure composed of methoxybenzenesulfonic acid amide, on the outermost surface of the antithrombogenic material can be determined by time-of-flight secondary ion mass spectrometry (hereinafter referred to as "TOF-SIMS").

[Measurement Conditions]

Apparatus: TOF.SIMS5 (manufactured by ION-TOF GmbH)
Primary ion species: $Bi_3^{++}$
Secondary ion polarity: positive and negative
Mass range (m/z): 0 to 1500
Raster size: 300 μm × 300 μm
Pixel number (each side): 256 pixels
Post-acceleration: 10kV
Measured degree of vacuum (before sample injection): $4 \times 10^{-7}$ Mpa
Primary ion acceleration voltage: 25 kV
Pulse width: 10.5 ns
Bunching: Yes (high mass resolution measurement)
Charge neutralization: Yes

The outermost surface of the antithrombogenic material herein means the portion from the measurement surface to a depth of 1 to 3 nm under the measurement conditions of TOF-SIMS.

Pulsed primary ions are radiated to the outermost surface of the antithrombogenic material placed in an ultrahigh vacuum, and then secondary ions released from the outermost surface of the antithrombogenic material, having a certain amount of kinetic energy, are introduced to the time-of-flight mass spectrometer. Since a mass spectrum is obtained depending on the mass of the secondary ions, organic substances and inorganic substances present on the outermost surface of the antithrombogenic material can be identified, and information on the abundance of each substance can be obtained based on its peak intensity.

More specifically, the skeletal structure composed of ethylene glycol or propylene glycol on the outermost surface of the antithrombogenic material can be confirmed based on at least one kind of peak selected from the group consisting of the $^{45}C_2H_5O^+$ peak, $^{59}C_3H_7O^+$ peak, $^{73}C_3H_5O_2^+$ peak, and $^{87}C_4H_7O_2^+$ peak of positive secondary ions found in TOF-SIMS.

The skeletal structure composed of 4-(aminomethyl)benzenecarboxyimidamide on the outermost surface of the antithrombogenic material can be confirmed based on at least one kind of peak selected from the group consisting of the $^{106}C_7H_8N^+$ peak, $^{117}C_7H_5N_2^+$ peak, $^{134}C_8H_{10}N_2^+$ peak, and $^{148}C_8H_{10}N_3^+$ peak of positive secondary ions, and the $^{119}C_7H_7N_2^-$ peak of negative secondary ions, found in TOF-SIMS. The skeletal structure composed of benzene amidine can be confirmed based on the $^{119}C_7H_7N_2^-$ peak of negative secondary ions found in TOF-SIMS. The skeletal structure composed of methoxybenzenesulfonic acid amide can be confirmed based on at least one kind of peak selected from the group consisting of the $^{117}C_7H_7SO_3^+$ peak of positive secondary ions, and the $^{64}SO_2^-$ peak, $^{171}C_7H_7SO_3^-$ peak, $^{186}C_7H_8SNO_3^-$ peak, and $^{212}C_9H_{10}SNO_3^-$ peak of negative secondary ions.

The presence of the betaine compound on the outermost surface of the antithrombogenic material can be confirmed based on at least one kind of peak selected from the group consisting of the $^{94}CH_2SO_3^-$ peak, $^{150}C_4H_8NO_3^-$ peak, and $^{166}C_5H_{12}NSO_3^-$ peak of negative secondary ions found in TOF-SIMS.

For example, in cases where the cationic polymer is PEI, the presence of the PEI on the outermost surface of the antithrombogenic material can be confirmed based on at least one kind of peak selected from the group consisting of the $^{18}N_4^+$ peak, $^{28}CH_2N^+$ peak, $^{43}CH_3N_2^+$ peak, and $^{70}C_4H_8N^+$ peak of positive secondary ions, and the $^{26}CN^-$ peak and $^{42}CNO^-$ peak of negative secondary ions, found in TOF-SIMS.

For example, in cases where the anionic polymer is PAA, the presence of the PAA on the outermost surface of the antithrombogenic material can be confirmed based on the $^{71}C_3H_3O_2^-$ peak of negative secondary ions found in TOF-SIMS.

For example, in cases where the base material is PET, the presence of the PET can be confirmed based on at least one kind of peak selected from the group consisting of the $^{76}C_6H_4^+$ peak, $^{104}C_7H_4NO^+$ peak, $^{105}C_7H_5O^+$ peak, and $^{149}C_8H_5O_3^+$ peak of positive secondary ions, and the $^{76}C_6N_4^-$ peak, $^{120}C_7H_4O_2^-$ peak, $^{121}C_7H_5O_2^-$ peak, $^{147}C_9H_7O_2^-$ peak, and $^{165}C_8H_5O_4^-$ peak of negative secondary ions, found in TOF-SIMS.

In the present invention, in cases where the anionic polymer is PAA, there are preferred ranges of the abundance ratios of the skeletal structure composed of 4-(aminomethyl)benzenecarboxyimidamide or benzene amidine and the skeletal structure composed of methoxybenzenesulfonic acid amide to PAA on the surface of the antithrombogenic material. In cases where the presence of PAA is confirmed based on the $^{71}C_3H_3O_2^-$ peak of negative secondary ions found in TOF-SIMS, and the presence of the skeletal structure composed of 4-(aminomethyl)benzenecarboxyimidamide or benzene amidine is confirmed based on the $^{119}C_7H_7N_2^-$ peak of negative secondary ions found in TOF-SIMS, the peak ratio of $^{119}C_7H_7N_2^-$ peak /$^{71}C_3H_3O_2^-$ peak is preferably not less than 0.05. In cases where the presence of PAA is confirmed based on the $^{71}C_3H_3O_2^-$ peak of negative secondary ions found in TOF-SIMS, and the presence of the skeletal structure composed of methoxybenzenesulfonic acid amide is confirmed based on the $^{64}SO_2$- peak, $^{171}C_7H_7SO_3^-$ peak, and $^{186}C_7H_8SNO_3^-$ peak of negative secondary ions found in TOF-SIMS, the peak ratio of $^{64}SO_2^-$ peak/$^{71}C_3H_3O_2^-$ peak is preferably not less than 0.6; the peak ratio of $^{171}C_7H_7SO_3^-$ peak/$^{71}C_3H_3O_2^-$ peak is preferably not less than 1.1; and

the peak ratio of $^{186}C_7H_8SNO_3^-$ peak/$^{71}C_3H_3O_2^-$ peak is preferably not less than 0.5.

In the present invention, the present inventors intensively studied to achieve maintenance of high antithrombogenicity for a longer period using an antithrombogenic compound other than heparin or heparin derivatives while elution of the compound is suppressed, compared to cases where the coating material is covalently bound by radiation irradiation. As a result, it was discovered that there are optimal values of the peak abundance ratio of the carbonyl-derived component in the C1s peak as measured by XPS on the surface of the antithrombogenic material.

That is, in the present invention, it was found that the peak abundance ratio of the carbonyl-derived component in the total component of the C1s peak (peak derived from carbon atoms) as measured by XPS on the surface of the antithrombogenic material is preferably not less than 1.0 atomic percent. In the coating material and the base material in the present invention, in cases where the peak abundance ratio of the carbonyl-derived component in the total component of the C1s peak (peak derived from carbon atoms) as measured by XPS on the surface of the antithrombogenic material is not less than 1.0 atomic percent, a sufficient amount of the coating material in the present invention is immobilized on the base material through covalent bonds, so that high antithrombogenicity can be maintained for a longer period compared to cases where the coating material is covalently bound by radiation irradiation.

More specifically, the peak abundance ratio of the carbonyl-derived component on the surface of the antithrombogenic material can be determined by XPS.

[Measurement Conditions]

Apparatus: ESCALAB 220iXL (manufactured by VG Scientific)
Excitation X-ray: monochromatic AlK $\alpha$1, 2 ray (1486.6 eV)
X-ray diameter: 1 mm
X-electron escape angle: 90° (the angle of the detector with respect to the surface of the antithrombogenic material)

The surface of the antithrombogenic material herein means the portion from the measurement surface to a depth of 10 nm as detected under the measurement conditions in XPS wherein the X-electron escape angle, that is, the angle of the detector with respect to the surface of the antithrombogenic material, is 90°. In the present invention, the base material may or may not contain nitrogen atoms.

By radiating X-ray to the surface of the antithrombogenic material, and measuring the energy of photoelectrons generated therefrom, the binding energy values of bound electrons in the substance can be determined. From the binding energy values, information on the atoms on the surface of the antithrombogenic material can be obtained, and, from the energy shift of the peak at each binding energy value, information on the valence and the binding state can be obtained. In addition, by using the peak area ratio of each peak, quantification, that is, calculation of the abundance ratios of each kind of atoms, valence, and binding state, is possible.

More specifically, the C1s peak, which indicates the abundance of carbon atoms with respect to the abundance of total atoms, appears near a binding energy value of 282 to 292 eV. The C1s peak can be mainly split into the c1 component (near 285 eV), which is attributed to carbon-hydrogen (hereinafter referred to as "CHx") bonds suggesting the presence of a saturated hydrocarbon(s) and/or the like, to carbon-carbon (hereinafter referred to as "C-C") bonds, and/or to carbon=carbon (hereinafter referred to as "C=C") bonds; the c2 component (near 286 eV), which is attributed to carbon-oxygen (hereinafter referred to as "C-O") bonds suggesting the presence of an ether(s) and/or hydroxyl groups, and/or to carbon-nitrogen (hereinafter referred to as "C-N") bonds; the c3 component (near 287 to 288 eV), which is attributed to carbon=oxygen (hereinafter referred to as "C=O") bonds suggesting the presence of carbonyl groups; the c4 component (near 288 to 289 eV), which is attributed to oxygen=carbon-oxygen (hereinafter referred to as "O=C-O") bonds suggesting the presence of ester groups and/or carboxyl groups; and the c5 component (near 290 to 292 eV), which is attributed to $\pi$-$\pi$* satellite peak (hereinafter referred to as "$\pi$-$\pi$") bonds suggesting the presence of a conjugated system(s) such as benzene rings. The abundance ratio of each split peak component can be calculated according to the Equation 1 below. In this calculation, the abundance ratio of carbon atoms and the abundance ratio of each split peak component to the abundance of total atoms are rounded to one decimal place.

$$\mathrm{Split}_{\mathrm{ratio}} = \mathrm{C1s}_{\mathrm{ratio}} \times (\mathrm{Split}_{\mathrm{percent}} / 100) \ldots \text{ Equation 1}$$

$\mathrm{Split}_{\mathrm{ratio}}$: abundance ratio of each split peak component (%)
$\mathrm{C1s}_{\mathrm{ratio}}$: abundance ratio of carbon atoms to the abundance of total atoms (%)

$Split_{percent}$: ratio of each split peak component in the total component of the C1s peak (%)

The c3 component, which is attributed to C=O bonds, obtained by splitting the C1s peak indicates the presence of carbonyl groups in the present invention. The ratio of the c3 component in the total component of the C1s peak, that is, the peak abundance ratio of the carbonyl-derived component in the total component of the C1s peak as measured by XPS on the surface of the antithrombogenic material in the present invention, is preferably not less than 1.0 atomic percent, more preferably not less than 2.0 atomic percent, still more preferably not less than 3.0 atomic percent. In cases where the peak abundance ratio of the carbonyl-derived component is less than 1.0 atomic percent, the number of covalent bonds due to carbonyl-derived amide bonds between the coating material and the base material is small, and therefore the coating amount of the coating material is small, so that the antithrombogenicity of interest cannot be obtained.
The N1s peak, which indicates the presence of nitrogen atoms, appears near a binding energy value of 396 eV to 403 eV. In the present invention, it was discovered that the area ratio of the N1s peak in the total peak component is preferably 1.0 to 12.0 atomic percent, more preferably 2.0 to 11.0 atomic percent, still more preferably 3.0 to 10.0 atomic percent. In the calculation of the abundance ratio of nitrogen atoms to the abundance of total atoms, the obtained value is rounded to one decimal place.
The antithrombogenic material of the present invention can be favorably used for medical equipments and medical instruments (artificial kidneys, artificial lungs, artificial blood vessels, artificial valves, stents, stent-grafts, catheters, free-thrombus capture devices, angioscopes, sutures, blood circuits, tubes, cannulae, blood bags, syringes, and the like). The antithrombogenic material of the present invention is especially preferably used as a material for free-thrombus capture devices and artificial blood vessels.
In cases where the antithrombogenic material of the present invention is used for a free-thrombus capture device, it is preferred to use the antithrombogenic material of the present invention for all constituents of the free-thrombus capture device. Since the porous material, which is the constituent for capturing free thrombi, requires highest antithrombogenicity, at least the porous material as the base material may be coated with the coating material. Examples of the porous material as the base material include, but are not limited to, porous membranes and meshes. Meshes are preferred since they have better uniformity of pores or apertures. Preferred examples of the material of the porous material include, but are not limited to, metals such as nickel-titanium alloy, and polyurethanes and polyesters. PET, which is a polyester, is more preferably used.
From the viewpoint of increasing the accuracy of capturing free thrombi, in cases where the mesh as the material is PET, the single fiber diameter of the fibers constituting the mesh is preferably 10 μm to 50 μm, more preferably 20 μm to 40 μm. The mesh aperture is preferably 10 μm to 200 μm, more preferably 50 μm to 150 μm.
In cases where the antithrombogenic material of the present invention is used for an artificial blood vessel, it is preferred to use the antithrombogenic material of the present invention for all constituents of the artificial blood vessel. Since the inner surface of the artificial blood vessel is in contact with blood, and therefore requires highest antithrombogenicity, at least the inner surface of the artificial blood vessel as the base material may be coated with the coating material. Preferred examples of the structure constituting the inner surface of the artificial blood vessel as the base material include, but are not limited to, fabric structures composed of warp and weft yarns constituted by monofilaments or multifilaments. Preferred examples of the material of the base material include, but are not limited to, nylons and polyesters, and ePTFE. PET, which is a polyester, is more preferably used.
From the viewpoint of achieving favorable flexibility of the artificial blood vessel, in cases where the mesh as the material is PET, monofilaments and multifilaments having a single fiber diameter of not more than 15 μm are preferred; monofilaments and multifilaments having a single fiber diameter of not more than 10 μm are more preferred; and monofilaments and multifilaments having a single fiber diameter of not more than 5 μm are still more preferred.
In cases of a conventional antithrombogenic material, coating of the mesh as the base material with a coating material may cause destruction of the microstructure of the mesh, apertures, leading to a decrease in the accuracy of capturing thrombi. Moreover, destruction of the microstructure of the inner surface of the artificial blood vessel, that is, the fabric structure composed of warp and weft yarns, may affect blood blow and/or the like to promote thrombus formation.
In cases where the thickness of the coating material with which the base material is coated is too large, the microstructure of the surface of the base material may be destroyed. Therefore, the coating material is preferably present on the surface of the antithrombogenic material with a thickness within the range of 1 to 600 nm. The thickness is more preferably 5 to 500 nm, still more preferably 10 to 400 nm. The thickness of the coating material herein means, for example, the distance from the start point to the end point of the range where atoms derived from the coating material can be observed using the later-mentioned scanning transmission electron microscope (hereinafter referred to as "STEM"), along the direction vertical to the surface of the base material. The thickness is determined as a mean of the thickness values measured at at least three positions. The term "present on the surface" means the presence of the coating material in the range including both the range extending from the surface of the base material toward the outside and the range extending from the surface of the base material toward the depth direction of the base material.
The method for producing the antithrombogenic material of the present invention is described below. For

example, in preparation of fibers constituting the mesh as the base material of a free-thrombus capture device, or in preparation of fibers constituting the fabric structure as the base material of an artificial blood vessel, the base material of interest may be added to a solution containing the whole coating material including the skeletal structure composed a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; the skeletal structure composed of 4-(aminomethyl)benzenecarboxyimidamide or benzene amidine; and the skeletal structure composed of methoxybenzenesulfonic acid amide; to perform the coating. Alternatively, the surface of the base material may be preliminarily coated with any one of the skeletal structures, followed by coating of the surface of the base material with other skeletal structures.

The solution containing the whole coating material may also contain a betaine compound.

In particular, from the viewpoint of maintaining high antithrombogenicity on the surface of the base material for a long period, a method in which the coating material containing: the skeletal structure composed a polymer containing, as a constituent monomer, a compound selected from the group consisting of ethylene glycol, propylene glycol, vinylpymolidone, vinyl alcohol, vinylcaprolactam, vinyl acetate, styrene, methyl methacrylate, hydroxyethyl methacrylate, and siloxane; the skeletal structure composed of 4-(aminomethyl)benzenecarboxyimidamide or benzene amidine; and the skeletal structure composed of methoxybenzenesulfonic acid amide; is covalently bound to the surface of the base material in a first coating step is more preferred.

The solution containing the whole coating material may also contain a betaine compound. In such a case, covalent bonding of the betaine compound may be carried out at the same time as covalent bonding of the coating material. Alternatively, covalent bonding of the betaine compound to the base material may be carried out after covalent bonding of the coating material to the base material. The betaine compound may be covalently bound to the base material through the later-mentioned cationic polymer, anionic polymer, and/or anionic compound.

The method for covalent bonding of the coating material to the surface of the base material is not limited. In cases where the base material has a functional group(s) (for example, hydroxyl, thiol, amino, carboxyl, aldehyde, isocyanate, and/or thioisocyanate), the base material may be covalently bound to the coating material by chemical reaction. For example, in cases where the surface of the base material has a carboxyl group and/or the like, a coating material having a hydroxyl group, thiol group, amino group, and/or the like may be covalently bound to the surface of the base material. Examples of the method of covalent bonding in such cases include a method in which a compound having a hydroxyl group, thiol group, amino group, and/or the like is covalently bound to the coating material, and the resulting coating material is covalently bound to the surface of the base material having a carboxyl group and/or the like.

As described later, in the present invention, the coating material which is an antithrombogenic compound other than heparin or heparin derivatives can be covalently bound to the surface of the base material without decreasing the antithrombin activation capacity, because the functional group(s) described above is contained in the coating material.

In cases where the base material does not have a functional group, examples of the method of covalent bonding include a method in which the surface of the base material is treated with plasma, corona, or the like, followed by covalent bonding of the coating material to the surface, and a method in which radiation imadiation is performed to cause generation of radicals on the surface of the base material and the coating material, and covalent bonding between the surface of the base material and the coating material is achieved by recombination reaction of the radicals. However, since the radicals generated by the radiation irradiation change especially the skeletal structure composed of 4-(aminomethyl)benzenecarboxyimidamide or benzene amidine and the skeletal structure composed of methoxybenzenesulfonic acid amide in the coating material, leading to a decrease in the antithrombin activation capacity. Therefore, the method in which the surface of the base material is treated with plasma, corona, or the like, followed by covalent bonding of the coating material to the surface, is more preferred.

In the present invention, from the viewpoint of maintaining high antithrombogenicity for a longer period, a first preceding step in which an anionic polymer comprising, as a constituent monomer, a compound selected from the group consisting of acrylic acid, methacrylic acid, $\alpha$-glutamic acid, $\gamma$-glutamic acid, and aspartic acid; or an anionic compound selected from the group consisting of oxalic acid, malonic acid, succinic acid, fumaric acid, glutaric acid, adipic acid, pimelic acid, suberic acid, azelaic acid, sebacic acid, malic acid, tartaric acid, and citric acid; is covalently bound to the surface of the base material is preferably carried out before the coating step. More preferably, a first preceding step in which a cationic polymer comprising, as a constituent monomer, a compound selected from the group consisting of alkyleneimines, vinylamines, allylamines, lysine, protamine, and diallyldimethylammonium chloride is bound to the surface of the base material is carried out, and then a second preceding step in which an anionic polymer or an anionic compound is covalently bound to the cationic polymer is carried out, followed by carrying out a coating step in which the coating material is covalently bound to the cationic polymer.

Preferably, in the present invention, from the viewpoint of maintaining high antithrombogenicity for a longer period, a betaine compound represented by General Formula (IX) or (X) is covalently bound to any one of the cationic polymer, anionic polymer, and anionic compound before the first preceding step or the second preceding step, or a betaine compound represented by General Formula (IX) or (X) is covalently bound to any one of the cationic polymer,

anionic polymer, and anionic compound after the coating step.

In cases where a polyester polymer is used as the material of the base material, the coating material may be brought into contact therewith under heat to allow covalent bonding by aminolysis reaction, although the method of covalent bonding is not limited thereto. Alternatively, ester bonds on the surface of the base material may be hydrolyzed by acid or alkali treatment, and carboxyl groups generated on the surface of the base material may be covalently bound to amino groups in the coating material by condensation reaction. In these methods, the reaction may be carried out by bringing the coating material into contact with the surface of the base material, or by bringing a solution of the coating material in a solvent into contact with the surface of the base material. Preferred examples of the solvent include water and alcohols. From the viewpoint of ease of handling, residual performance, and the like, water is especially preferred.

It was found, in the present invention, that a step of hydrolyzing and oxidizing ester bonds on the surface of the base material before the coating step is important. More specifically, a method in which treatment is carried out using an acid or an alkali, as well as an oxidizing agent, is preferably used. It was found, in the present invention, that the surface of the base material cannot be coated with a sufficient amount of the coating material by a method in which treatment is carried out with only an acid or an alkali. This is because, for example, in the method in which treatment is carried out using only an acid or an alkali, hydroxyl groups and carboxyl groups generated by hydrolysis of the ester bonds coexist, resulting in inefficient progress of the condensation reaction with amino groups in the coating material. Moreover, the presence of hydroxyl groups is not preferred since they are likely to activate complement when they are in contact with blood. That is, from the viewpoint of increasing antithrombogenicity by increasing the coating amount of the coating material without activating complement, the method in which treatment is carried out using an acid or an alkali, as well as an oxidizing agent, is especially preferably used.

In terms of the combination in the step of hydrolyzing and oxidizing ester bonds on the surface of the base material using an acid or an alkali, as well as an oxidizing agent in the present invention, it was discovered that a method in which treatment is carried out using an acid and an oxidizing agent is most preferred. The treatment using an acid and an oxidizing agent may be carried out after treating the surface of the base material using an alkali.

Examples of the type of the acid used include, but are not limited to, inorganic acids such as hydrochloric acid, hydrobromic acid, hydroiodic acid, hypochlorous acid, chlorous acid, perchloric acid, sulfuric acid, fluorosulfonic acid, nitric acid, phosphoric acid, hexafluoroantimonic acid, tetrafluoroboric acid, chromic acid, and boric acid; sulfonic acids such as methanesulfonic acid, ethanesulfonic acid, benzenesulfonic acid, *p*-toluenesulfonic acid, trifluoromethanesulfonic acid, and sodium polystyrene sulfonate; carboxylic acids such as acetic acid, citric acid, formic acid, gluconic acid, lactic acid, oxalic acid, and tartaric acid; vinyl carboxylic acids such as ascorbic acid and Meldrum's acid; and nucleic acids such as deoxyribonucleic acid and ribonucleic acid. Among these, hydrochloric acid, sulfuric acid, and the like are more preferred from the viewpoint of, for example, ease of handling.

Examples of the type of the base used include, but are not limited to, hydroxides of alkali metals such as lithium hydroxide, sodium hydroxide, potassium hydroxide, rubidium hydroxide, and cesium hydroxide; hydroxides of tetraalkylammonium such as tetramethylammonium hydroxide and tetraethylammonium hydroxide; hydroxides of alkaline earth metals such as calcium hydroxide, strontium hydroxide, barium hydroxide, europium hydroxide, and thallium hydroxide; guanidine compounds; hydroxides of ammine complexes such as diammine silver (I) hydroxide and tetraammine copper (II) hydroxide; trimethylsulfonium hydroxide; and diphenyliodonium hydroxide. Among these, lithium hydroxide, sodium hydroxide, potassium hydroxide, and the like are more preferred from the viewpoint of, for example, ease of handling.

Examples of the type of the oxidizing agent used include, but are not limited to, potassium nitrate; hypochlorous acid; chlorous acid; perchloric acid; halogens such as fluorine, chlorine, bromine, and iodine; permanganates such as potassium permanganate, sodium permanganate trihydrate, ammonium permanganate, silver permanganate, zinc permanganate hexahydrate, magnesium permanganate, calcium permanganate, and barium permanganate; ceric ammonium nitrate; chromic acid; dichromic acid; peroxides such as hydrogen peroxide solution; Tollens' reagent; and sulfur dioxide. Among these, permanganates are more preferred from the viewpoint of, for example, their strength as oxidizing agents and favorable prevention of deterioration of the antithrombogenic material.

Examples of the method for covalently binding the coating material to the surface of the base material include a method in which condensation reaction is carried out using a dehydration-condensation agent or the like.

Examples of the type of the dehydration-condensation agent used include, but are not limited to, carbodiimide compounds such as *N,N*'-dicyclohexyl carbodiimide, *N,N*'-diisopropyl-carbodiimide, 1-ethyl-3-(3-dimethylaminopropyl)carbodiimide, 1-ethyl-3-(3-dimethylaminopropyl)carbodiimide hydrochloride (hereinafter referred to as "EDC"), 1,3-bis(2,2-dimethyl-1,3-dioxolan-4-ylmethyl)carbodiimide, *N*-{3-(dimethylamino)propyl-}-*N*'-ethylcarbodiimide, *N*-{3-(dimethylamino)propyl-}-*N*'-ethylcarbodiimide methiodide, *N*-*tert*-butyl-*N'*-ethylcarbodiimide, *N*-cyclohexyl-*N*'-(2-morpholinoethyl)carbodiimide, *meso*-*p*-toluenesulfonate, *N,N'*-di-*tert*-butylcarbodiimide, and *N,N'*-di-*p*-tricarbodiimide; and triazine compounds such as 4-(4,6-dimethoxy-1,3,5-triazin-2-yl)-4-methylmorpholinium chloride n-hydrate (hereinafter referred to as "DMT-MM").

The dehydration-condensation agent may be used together with a dehydration-condensation promoter. Examples of the dehydration-condensation promoter used include, but are not limited to, pyridine, 4-dimethylaminopyridine

(hereinafter referred to as "DMAP"), triethylamine, isopropylamine, 1-hydroxybenzotriazole, and *N*-hydroxysuccinimide.

The coating material, dehydration-condensation agent, and dehydration-condensation promoter may be prepared as a mixed aqueous solution to be used for the reaction, or may be sequentially added to perform the reaction.

In cases where ePTFE is used as a material of the base material, a method in which the surface of the base material is functionalized using plasma or corona may be used, although the method is not limited thereto. Alternatively, a method in which a fluorocarbon-resin surface treatment agent or the like is used for extraction of fluorine atoms present on the surface of the base material, and hydroxyl groups, carboxyl groups, carbonyl groups, and/or the like are formed by reaction with oxygen, hydrogen, water vapor, and/or the like in the air, may be used.

In the same manner as in the cases of the polyester polymer base material, the coating step of covalently binding the coating material, cationic polymer, and anionic polymer or anionic compound, to the surface of the ePTFE base material, and the first preceding step and the second preceding step, may be carried out.

In the present invention, the anti-platelet adhesion capacity and the antithrombin activation capacity were used as indices of antithrombogenicity.

The anti-platelet adhesion capacity is an index indicating the degree of inhibition of platelet adhesion on the surface of the antithrombogenic material. The anti-platelet adhesion capacity can be measured based on the platelet adhesion rate described in the later-mentioned Evaluation 1. The platelet adhesion rate is preferably less than 1.5%, more preferably less than 1.0%, still more preferably less than 0.05%, still more preferably less than 0.03%.

The antithrombin activation capacity is an index indicating the degree of inhibition of thrombin activation on the surface of the antithrombogenic material. The antithrombin activation capacity can be measured based on the thrombin activation ratio described in the later-mentioned Evaluation 2. The thrombin activation ratio is preferably less than 40%, more preferably less than 30%, still more preferably less than 20%, still more preferably less than 8%.

Preferably, in the antithrombogenic material of the present invention, the base material is coated with the coating material not from the interface of the base material, but the coating material is also present in the depth direction from the interface of the base material.

More specifically, whether or not the coating material is present in the depth direction from the interface of the base material can be confirmed by combination of, for example, a STEM and XPS. A STEM has detectors such as an energy dispersive X-ray spectrometer (hereinafter referred to as "EDX") and an electron energy-loss spectrometer (hereinafter referred to as "EELS"). Measurement conditions for the STEM are as follows.

[Measurement Conditions]

Apparatus: field emission transmission electron microscope JEM-2100F (manufactured by JEOL Ltd.)
EELS detector: GIF Tridiem (manufactured by GATAN, Inc.)
EDX detector: JED-2300T (manufactured by JEOL Ltd.)
Image acquisition: Digital Micrograph (manufactured by GATAN, Inc.)
Sample preparation: ultrathin sectioning (suspension using a copper microgrid; use of an acrylic resin as an embedding resin)
Acceleration voltage: 200 kV
Beam diameter: 0.7-nm diameter
Energy resolution: about 1.0 eV FWHM

The surface of the antithrombogenic material herein means the portion from the measurement surface to a depth of 10 nm as measured by XPS, and the interface of the antithrombogenic material herein means the border with the acrylic resin in which the antithrombogenic material is embedded during the sample preparation before the measurement using the STEM.

Whether or not the coating material is present in the depth direction from the interface of the base material can be confirmed by, for example, measurement using the STEM. The presence of the coating material can be confirmed by carrying out observation of atoms derived from the coating material from the interface toward the depth direction of the antithrombogenic material, and finding atoms derived from the coating material at a position deeper than a position where atoms derived from the base material are found. For example, in cases where the base material is a polyester or ePTFE, the presence of the coating material can be confirmed by finding sulfur atoms derived from the coating material at a position deeper than a position where oxygen atoms, fluorine atoms, and/or the like derived from the base material are found. The interface of the base material herein means the position in the depth direction where the atoms derived from the base material are found. The presence of each kind of atoms is judged based on whether a peak intensity derived from the atoms can be found in a spectrum obtained by STEM measurement after subtraction of the background.

In the present invention, when the atomic distribution is observed from the position of the interface of the base

material toward the depth direction thereof, that is, from the position where atoms derived from the base material are present rather than from the surface of the antithrombogenic material toward the depth direction, atoms derived from the coating material are present at positions more distant from the interface of the antithrombogenic material. More specifically, atoms derived from the coating material are preferably present to a depth of at least 15 to 200 nm, more preferably present to a depth of 20 to 100 nm, still more preferably present to a depth of 50 to 90 nm, from the interface of the base material. It was found, in the present invention, that, in cases where the coating material is present to a depth of at least 15 to 200 nm from the interface of the base material, the coating material containing: a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; a skeletal structure composed of 4-(aminomethyl)benzenecarboxyimidamide or benzene amidine; and a skeletal structure composed of methoxybenzenesulfonic acid amide; continuously exhibits high antithrombogenicity for a long period. Similarly, atoms derived from the betaine compound are preferably present to a depth of 15 to 200 nm, more preferably present to a depth of 20 to 100 nm, still more preferably present to a depth of 50 to 90 nm, from the surface of the base material. In cases where the coating material is present to a depth of only less than 15 nm, the coating amount of the coating material is small, which is not preferred. On the other hand, in cases where the coating material is present to a depth of more than 200 nm, high antithrombogenicity can be maintained for a long period, but deterioration of the polyester base material due to hydrolysis caused by the acid or the alkali, and the oxidizing agent, rapidly proceeds, leading to a decrease in mechanical properties of the base material such as the tensile strength, which is not preferred. In the present invention, in cases of a base material which is not a porous material or the like having pores, the coating material is preferably bound to a depth of 15 to 200 nm, more preferably bound to a depth of 50 to 100 nm.

EXAMPLES

The present invention is described below in detail by way of Examples and Comparative Examples. However, the present invention is not limited thereto.

(Example 1)

A PET mesh (diameter, 27 μm; interfiber distance, 100 μm) as a base material was immersed in an aqueous solution of 5.0 wt% potassium permanganate (manufactured by Wako Pure Chemical Industries, Ltd.) and 0.6 mol/L sulfuric acid (manufactured by Wako Pure Chemical Industries, Ltd.), and the reaction was allowed to proceed at 60°C for 3 hours, thereby hydrolyzing and oxidizing the PET mesh (hydrolysis/oxidation step). The aqueous solution after the reaction was removed, and the mesh was washed with 6 N hydrochloric acid (manufactured by Wako Pure Chemical Industries, Ltd.) and distilled water.

Subsequently, the PET mesh was immersed in an aqueous solution of 1.0 wt% Compound A (the General Formula (XI) shown below) which is a part of the coating material, and 2 molar equivalents of sodium hydroxide (manufactured by Wako Pure Chemical Industries, Ltd.) and 3 molar equivalents of DMT-MM (manufactured by Wako Pure Chemical Industries, Ltd.) with respect to Compound A, and the reaction was allowed to proceed at 30°C for 2 hours, thereby covalently binding Compound A to the PET mesh by condensation reaction (coating step ). The aqueous solution after the reaction was removed, and the mesh was washed with distilled water.

[Chemical Formula 18]

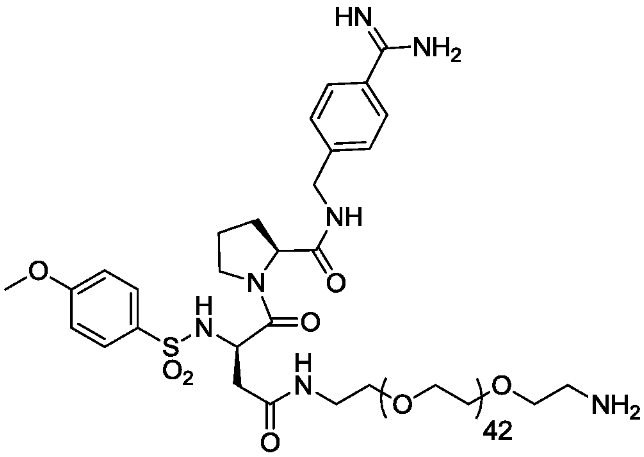

(XI)

The platelet adhesion rate and the thrombin activation ratio were evaluated. The results are shown in Table 1. As shown in Table 1, the platelet adhesion rate and the thrombin activation ratio were sufficiently low.

(Example 2)

The same operation as in Example 1 was carried out except that Compound B (the General Formula (XII) shown below) was used instead of Compound A, thereby covalently binding Compound B to the PET mesh by condensation reaction.

[Chemical Formula 19]

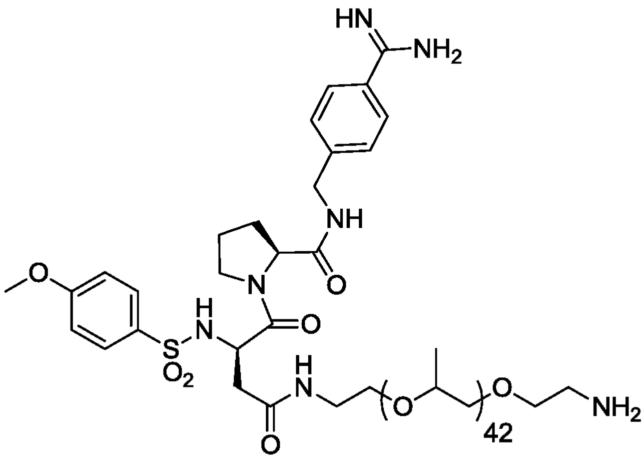

(XII)

The platelet adhesion rate and the thrombin activation ratio were evaluated. The results are shown in Table 1. As shown in Table 1, the platelet adhesion rate and the thrombin activation ratio were sufficiently low.

(Example 3)

The same operation as in Example 1 was carried out except that Compound C (the General Formula (XIII) shown below) was used instead of Compound A, thereby covalently binding Compound C to the PET mesh by condensation reaction.

[Chemical Formula 20]

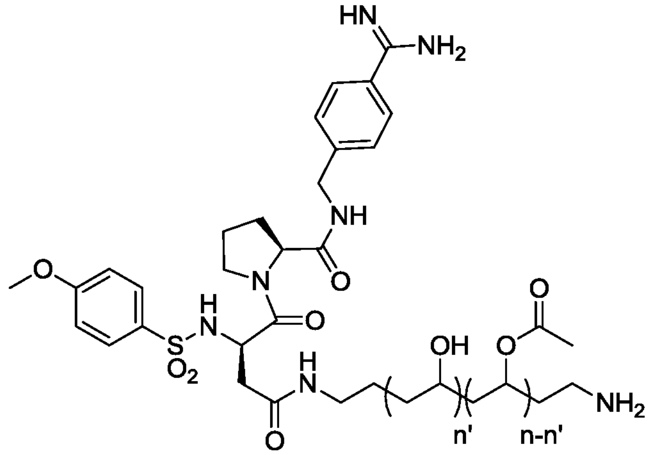

(XIII)

[wherein n=42, and the degree of saponification was 85 to 90%].

The platelet adhesion rate and the thrombin activation ratio were evaluated. The results are shown in Table 1. As shown in Table 1, the platelet adhesion rate and the thrombin activation ratio were sufficiently low.

(Example 4)

The same operation as in Example 1 was carried out except that Compound D (the General Formula (XIV) shown below) was used instead of Compound A, thereby covalently binding Compound D to the PET mesh by condensation reaction.

[Chemical Formula 21]

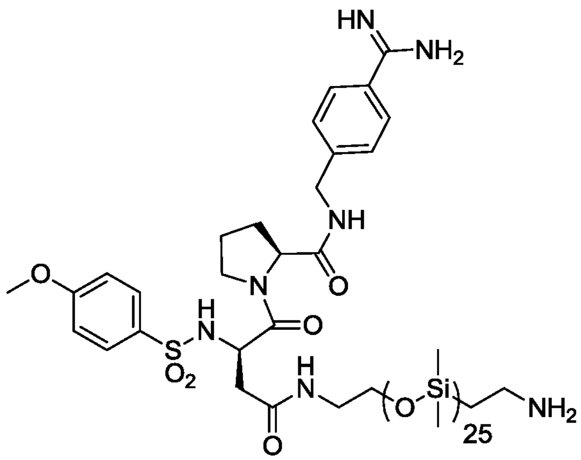

(XIV)

The platelet adhesion rate and the thrombin activation ratio were evaluated. The results are shown in Table 1. As shown in Table 1, the platelet adhesion rate and the thrombin activation ratio were sufficiently low.

(Example 5)

By the same operation as in Example 1, the PET mesh was hydrolyzed and oxidized, and the resulting PET mesh was immersed in an aqueous solution of 0.5 wt% DMT-MM and 5.0 wt% PEI (LUPASOL (registered trade mark) P, manufactured by BASF), followed by allowing the reaction to proceed at 30°C for 2 hours, thereby covalently binding PEI to the PET mesh by condensation reaction (first preceding step). The aqueous solution after the reaction was removed, and the mesh was washed with distilled water.

The PET mesh was then immersed in dimethylacetamide containing 0.5 wt% DMT-MM and 40 wt% succinic anhydride (manufactured by Wako Pure Chemical Industries, Ltd.), and the reaction was allowed to proceed at 50°C for 17 hours (second preceding step). The solution after the reaction was removed, and the mesh was washed with methanol and distilled water. Finally, Compound A was covalently bound to the PET mesh by condensation reaction. The aqueous solution after the reaction was removed, and the mesh was washed with distilled water.
The platelet adhesion rate and the thrombin activation ratio were evaluated. The results are shown in Table 1. As shown in Table 1, the platelet adhesion rate and the thrombin activation ratio were sufficiently low.

(Example 6)

The same operation as in Example 5 was carried out except that 0.5 wt% PAA (manufactured by Wako Pure Chemical Industries, Ltd.) was used instead of 40wt% succinic anhydride; the reaction was carried out at 30°C for 2 hours rather than at 50°C for 17 hours; and washing with an aqueous sodium carbonate solution and distilled water was carried out instead of washing with methanol and distilled water; thereby covalently binding Compound A by condensation reaction. The aqueous solution after the reaction was removed, and the mesh was washed with distilled water.
The platelet adhesion rate and the thrombin activation ratio were evaluated. The results are shown in Table 1. As shown in Table 1, the platelet adhesion rate and the thrombin activation ratio were sufficiently low.

(Example 7)

The same operation as in Example 6 was carried out except that Compound E (the General Formula (XV) shown below) was used instead of Compound A, thereby covalently binding Compound E to the PET mesh by condensation reaction. The aqueous solution after the reaction was removed, and the mesh was washed with distilled water.

[Chemical Formula 22]

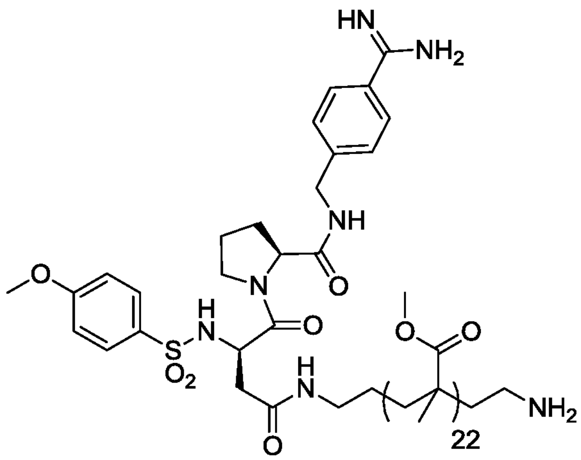

(XV)

The platelet adhesion rate and the thrombin activation ratio were evaluated. The results are shown in Table 1. As shown in Table 1, the platelet adhesion rate and the thrombin activation ratio were sufficiently low.

(Comparative Example 1)

A PET mesh was immersed in an aqueous solution of 1.0 wt% Compound A, and irradiated with 5 kGy γ-ray (JS-8500 Cobalt 60 γ-ray irradiator, manufactured by Nordion International Inc.) to allow covalent bonding (coating step). The aqueous solution after the reaction was removed, and the mesh was washed with Triton-X100 (manufactured by Sigma-Aldrich), physiological saline, and distilled water.
The platelet adhesion rate and the thrombin activation ratio were evaluated. The results are shown in Table 1. As shown in Table 1, the thrombin activation ratio was low, but the platelet adhesion rate was high.

(Comparative Example 2)

The same operation as in Comparative Example 1 was carried out except that Compound F (the General Formula (XVI) shown below) was used instead of Compound A, thereby covalently binding Compound F to the PET mesh. The aqueous solution after the reaction was removed, and the mesh was washed with Triton-X100 (manufactured by Sigma-Aldrich), physiological saline, and distilled water.

[Chemical Formula 23]

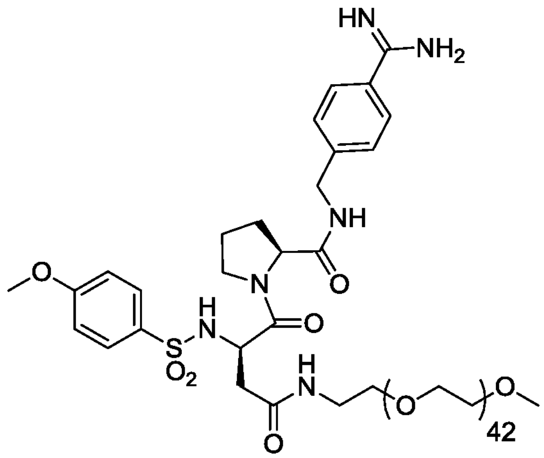

(XVI)

The platelet adhesion rate and the thrombin activation ratio were evaluated. The results are shown in Table 1. As shown in Table 1, the thrombin activation ratio was low, but the platelet adhesion rate was high.

(Comparative Example 3: Patent Document 12)

The same operation as in Comparative Example 1 was carried out except that Compound F and Compound G (copolymer of polyether and polysiloxane (F-3031, manufactured by Shin-Etsu Chemical Co., Ltd.)) were used instead of Compound A, thereby covalently binding Compound F and Compound G to the PET mesh. The aqueous solution after the reaction was removed, and the mesh was washed with Triton-X100 (manufactured by Sigma-Aldrich), physiological saline, and distilled water.

The platelet adhesion rate and the thrombin activation ratio were evaluated. The results are shown in Table 1. As shown in Table 1, the platelet adhesion rate was low, but the thrombin activation ratio was high. Thus, performances such as those observed in the Examples could not be achieved by Patent Document 12.

(Comparative Example 4)

The same operation as in Example 1 was carried out except that Compound H (the General Formula (XVII) shown below) was used instead of Compound A, thereby covalently binding Compound H to the PET mesh. The aqueous solution after the reaction was removed, and the mesh was washed with distilled water.

[Chemical Formula 24]

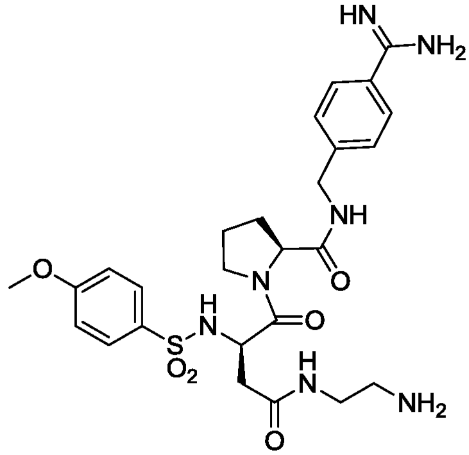

(XVII)

The platelet adhesion rate and the thrombin activation ratio were evaluated. The results are shown in Table 1. As shown in Table 1, the platelet adhesion rate and the thrombin activation ratio were high.

(Example 8)

A PET mesh (diameter, 27 μm; interfiber distance, 100 μm) as a base material was immersed in an aqueous solution of 5.0 wt% potassium permanganate (manufactured by Wako Pure Chemical Industries, Ltd.) and 0.6 mol/L sulfuric acid (manufactured by Wako Pure Chemical Industries, Ltd.), and the reaction was allowed to proceed at 60°C for 3 hours, thereby hydrolyzing and oxidizing the PET mesh (hydrolysis/oxidation step). The aqueous solution after the reaction was removed, and the mesh was washed with 6 N hydrochloric acid (manufactured by Wako Pure Chemical Industries, Ltd.) and distilled water.

Subsequently, the PET mesh was immersed in an aqueous solution of 0.5 wt% DMT-MM (manufactured by Wako Pure Chemical Industries, Ltd.) and 5.0 wt% PEI (LUPASOL (registered trade mark) P, manufactured by BASF), followed by allowing the reaction to proceed at 30°C for 2 hours, thereby covalently binding PEI to the PET mesh by condensation reaction (first preceding step). The aqueous solution after the reaction was removed, and the mesh was washed with distilled water.

The PET mesh was then immersed in an aqueous solution of 0.5 wt% DMT-MM and 0.5 wt% Compound I (the General Formula (XVIII) shown below), followed by allowing the reaction to proceed at 30°C for 2 hours (second preceding step). The solution after the reaction was removed, and the mesh was washed with an aqueous sodium carbonate solution and distilled water.

[Chemical Formula 25]

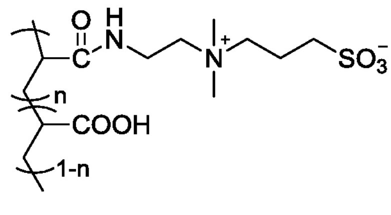

(XVIII)

[wherein n = 0.4].

Finally, the mesh was immersed in an aqueous solution of 2 molar equivalents of sodium hydroxide (manufactured by Wako Pure Chemical Industries, Ltd.) and 3 molar equivalents of DMT-MM (manufactured by Wako Pure Chemical Industries, Ltd.) with respect to 1.0 wt% Compound A, and the reaction was allowed to proceed at 30°C for 2 hours, thereby covalently binding Compound A to the PET mesh by condensation reaction (coating step). The aqueous solution after the reaction was removed, and the mesh was washed with distilled water.

The platelet adhesion rate and the thrombin activation ratio were evaluated. The results are shown in Table 1. As shown in Table 1, the platelet adhesion rate and the thrombin activation ratio were sufficiently low.

(Example 9)

The same operation as in Example 8 was carried out except that Compound B was used instead of Compound A, thereby covalently binding Compound B to the PET mesh by condensation reaction.
The platelet adhesion rate and the thrombin activation ratio were evaluated. The results are shown in Table 1. As shown in Table 1, the platelet adhesion rate and the thrombin activation ratio were sufficiently low.

(Example 10)

The same operation as in Example 8 was carried out except that Compound C was used instead of Compound A, thereby covalently binding Compound C to the PET mesh by condensation reaction.
The platelet adhesion rate and the thrombin activation ratio were evaluated. The results are shown in Table 1. As shown in Table 1, the platelet adhesion rate and the thrombin activation ratio were sufficiently low.

(Example 11)

The same operation as in Example 8 was carried out except that Compound D was used instead of Compound A, thereby covalently binding Compound D to the PET mesh by condensation reaction.
The platelet adhesion rate and the thrombin activation ratio were evaluated. The results are shown in Table 1. As shown in Table 1, the platelet adhesion rate and the thrombin activation ratio were sufficiently low.

(Example 12)

The same operation as in Example 8 was carried out except that PAA (manufactured by Wako Pure Chemical Industries, Ltd.) was used instead of Compound I, thereby covalently binding Compound A to the PET mesh by condensation reaction. Subsequently, the PET mesh was immersed in an aqueous solution of 0.5 wt% DMT-MM and 5.0 wt% Compound J (the General Formula (XIX) shown below), followed by allowing the reaction to proceed at 30°C for 2 hours. The solution after the reaction was removed, and the mesh was washed with distilled water.

[Chemical Formula 26]

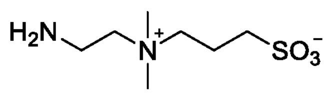

(XIX)

The platelet adhesion rate and the thrombin activation ratio were evaluated. The results are shown in Table 1. As shown in Table 1, the platelet adhesion rate and the thrombin activation ratio were sufficiently low.

(Example 13)

The same operation as in Example 12 was carried out except that Compound K (the General Formula (XX) shown below) was used instead of Compound J, thereby covalently binding Compound A and Compound K to the PET mesh by condensation reaction.

[Chemical Formula 27]

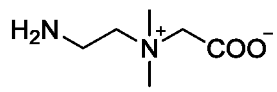

(XX)

The platelet adhesion rate and the thrombin activation ratio were evaluated. The results are shown in Table 1. As shown in Table 1, the platelet adhesion rate and the thrombin activation ratio were sufficiently low.

(Example 14)

The same operation as in Example 12 was carried out except that a solution of 40 wt% succinic anhydride

(manufactured by Wako Pure Chemical Industries, Ltd.) in dimethylacetamide (manufactured by Wako Pure Chemical Industries, Ltd.) was used instead of 0.5 wt% aqueous PAA solution; the reaction was carried out at 50°C for 17 hours rather than at 30°C for 2 hours; and washing with methanol and distilled water was carried out instead of washing with an aqueous sodium carbonate solution and distilled water; thereby covalently binding Compound A and Compound J to the PET mesh by condensation reaction.

The platelet adhesion rate and the thrombin activation ratio were evaluated. The results are shown in Table 1. As shown in Table 1, the platelet adhesion rate and the thrombin activation ratio were sufficiently low.

(Comparative Example 5)

The same operation as in Comparative Example 4 was carried out except that Compound H and Compound J were used instead of Compound H, thereby covalently binding Compound H and Compound J to the PET mesh.

The platelet adhesion rate and the thrombin activation ratio were evaluated. The results are shown in Table 1. As shown in Table 1, the platelet adhesion rate was lower than those in Comparative Examples 1 and 2, but the thrombin activation ratio was higher than those in Comparative Examples 1 and 2.

(Comparative Example 6)

The same operation as in Comparative Example 5 was carried out except that Compound L (aminoethyl polyethylene glycol (SUNBRIGHT ME-020EA, manufactured by NOF Corporation)) was used instead of Compound H, thereby covalently binding Compound L and Compound J to the PET mesh.

The platelet adhesion rate and the thrombin activation ratio were evaluated. The results are shown in Table 1. As shown in Table 1, the platelet adhesion rate was lower than those in Comparative Examples 1 and 2, but the thrombin activation ratio was higher than those in Comparative Examples 1 and 2.

In relation to the anti-platelet adhesion capacity and the antithrombin activation capacity of the antithrombogenic material of the present invention, the platelet adhesion rate and the thrombin activation ratio were evaluated by the following methods.

(Evaluation 1: Evaluation of Platelet Adhesion Rate)

Citrated human blood was centrifuged at 20°C at 1000 rpm for 15 minutes to prepare human platelet-rich plasma. The human platelet-rich plasma was then diluted with physiological saline (manufactured by Otsuka Pharmaceutical Factory, Inc.) to a platelet count of $1.2 \times 10^8$/mL, to obtain diluted human platelet-rich plasma. An antithrombogenic material (for example, PET mesh) coated with a coating material was cut into a size of $1.0 \times 1.0$ cm, and placed in a microtube. To the microtube, the diluted platelet liquid in an amount corresponding to a platelet count of $6.0 \times 10^7$ was added, and the microtube was then incubated at 37°C for 30 minutes with shaking. Further, 20 μL of adenosine diphosphate (manufactured by Sigma-Aldrich) was added thereto, and the microtube was then incubated at 37°C for 30 minutes with shaking. The mesh was removed from the microtube, and rinsed with PBS(-) (manufactured by Nissui Pharmaceutical Co., Ltd.). Thereafter, destruction of platelets was carried out according to the procedure described for the LDH Cytotoxicity Detection kit (manufactured by Takara Bio Inc.), and the activity of lactate dehydrogenase (hereinafter referred to as "LDH") produced as a result was measured. By reference to a separately prepared calibration curve, the number of adherent platelets was determined. The ratio of the number of platelets in contact with the mesh to the number of platelets in the diluted human platelet-rich plasma before the contact with the mesh was calculated according to the following Equation 2, to determine the platelet adhesion rate.

2, to determine the platelet adhesion rate.

$$\text{Platelet adhesion rate (\%)} = (\text{number of adherent platelets after contact} / \text{number of platelets in diluted human platelet-rich plasma}) \times 100 \ldots \text{Equation 2}$$

Here, the lower the measurement result for Evaluation 1, that is, the platelet adhesion rate, the better. The platelet adhesion rate is preferably less than 1.5%, more preferably not more than 0.05%.

(Evaluation 2: Evaluation of Thrombin Activation Ratio)

An antithrombogenic material (for example, PET mesh) coated with a coating material was cut into a size of

1.0 × 1.0 cm, and placed in a microtube. To the microtube, 500 μL of 0.1 U/mL thrombin (manufactured by Haematologic Technologies Inc.) solution prepared with PBS(-) was added, and the microtube was then incubated at 37°C for 30 minutes with shaking. Subsequently, 500 μL of 400 μmol/L S-2238 (manufactured by Sekisui Medical Co., Ltd.) solution prepared with PBS(-) was added thereto, and the microtube was then incubated at 37°C for 45 minutes with shaking. The absorbance at 405 nm was measured using a microplate reader (MTP-300, manufactured by Corona Electric Co., Ltd.), and the ratio of the absorbance of the mesh solution to the absorbance of a blank solution was calculated according to the following Equation 3, to determine the thrombin activation ratio.

according to the following Equation 3, to determine the thrombin activation ratio.

$$\text{Thrombin activation rate (\%)} = [(At / Ab)] \times 100 \ldots \text{Equation 3}$$

At: absorbance of mesh solution
Ab: absorbance of blank solution

Here, the lower the measurement result for Evaluation 2, that is, the thrombin activation rate, the better. The thrombin activation rate is preferably less than 40%, more preferably not more than 8%.

[Table 1]

| | Compound represented by General Formula (I) | Skeletal structure composed of polymer | Skeletal structure composed of 4-(aminomethyl)benzenecar boxyimidamide or benzene amidine | Skeletal structure composed of methoxybenzenesulfonic acid amide | Abundance ratio of carbonyl groups (atomic percent) | Type of cationic polymer | Type of anionic polymer | Type of betaine compound | Platelet adhesion rate (%) | Thrombin activation ratio (%) |
|---|---|---|---|---|---|---|---|---|---|---|
| Example 1 | Compound A | Polyethylene glycol | Present | Present | 3 | - | - | - | 0.31 | 13.9 |
| Example 2 | Compound B | Polypropylene glycol | Present | Present | 3 | - | - | - | 0.27 | 12.8 |
| Example 3 | Compound C | Polyvinyl alcohol | Present | Present | 3 | - | - | - | 0.22 | 11.6 |
| Example 4 | Compound D | Polysiloxane | Present | Present | 4 | - | - | - | 0.26 | 12.3 |
| Example 5 | Compound A | Polyethylene qlvcol | Present | Present | 17 | Polyethyleneimine | Succinic acid | - | 0.17 | 8.2 |
| Example 6 | Compound A | Polyethylene qlvcol | Present | Present | 13 | Polyethyleneimine | Polyacrylic acid | Polyacrylic acid - | 0.08 | 3.3 |
| Example 7 | Compound E | Polymethyl methacrylate | Present | Present | 14 | Polyethyleneimine | Polyacrylic acid | acid - | 0.06 | 2.9 |
| Comparative Example 1 | Compound A | Polyethylene glycol | Present | Present | <1 | - | - | - | 1.86 | 16.2 |
| Comparative Example 2 | Compound F | Polyethylene glycol | Present | Present | <1 | - | - | - | 1.78 | 11.7 |
| Comparative Example 3 | Compound F and Compound G | Polyethylene glycol and polysiloxane | Present | Present | <1 | - | - | - | 0.06 | 93.9 |
| Comparative Example 4 | Compound H | Absent | Present | Present | 2 | - | - | - | 2.40 | 48.0 |

(continued)

| | Compound represented by General Formula (I) | Skeletal structure composed of polymer | Skeletal structure composed of 4-(aminomethyl)benzenecar boxyimidamide or benzene amidine | Skeletal structure composed of methoxybenzenesulfonic acid amide | Abundance ratio of carbonyl groups (atomic percent) | Type of cationic polymer | Type of anionic polymer | Type of betaine compound | Platelet adhesion rate (%) | Thrombin activation ratio (%) |
|---|---|---|---|---|---|---|---|---|---|---|
| Example 8 | Compound A | Polyethylene glycol | Present | Present | 15 | Polyethyleneimine | Polyacrylic acid | Sulfobetaine | 0.03 | 3.6 |
| Example 9 | Compound B | Polypropylene glycol | Present | Present | 15 | Polyethyleneimine | Polyacrylic acid | Sulfobetaine | 0.03 | 4.2 |
| Example 10 | Compound C | Polyvinyl alcohol | Present | Present | 15 | Polyethyleneimine | Polyacrylic acid | Sulfobetaine | 0.02 | 3.8 |
| Example 11 | Compound D | Polysiloxane | Present | Present | 16 | Polyethyleneimine | Polyacrylic acid | Sulfobetaine | 0.02 | 3.9 |
| Example 12 | Compound A | Polyethylene qlvcol | Present | Present | 17 | Polyethyleneimine | Polyacrylic acid | Sulfobetaine | 0.05 | 3.1 |
| Example 13 | Compound A | Polyethylene qlvcol | Present | Present | 17 | Polyethyleneimine | Polyacrylic acid | Carboxvbetaine | 0.05 | 3.3 |
| Example 14 | Compound A | Polyethylene glycol | Present | Present | 19 | Polyethyleneimine | Succinic acid | Sulfobetaine | 0.05 | 7.9 |
| Comparative Example 5 | Compound H | Absent | Present | Present | 2 | - | - | Sulfobetaine | 1.08 | 72.1 |
| Comparative Example 6 | Compound L | Polyethylene glycol | Absent | Absent | 2 | - | - | Sulfobetaine | 0.96 | 98.8 |

From the data in Table 1, it is clear that covalent bonding of a coating material containing: a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; a skeletal structure composed of benzene amidine; and a skeletal structure composed of methoxybenzenesulfonic acid amide; to a base material decreases the platelet adhesion rate and the thrombin activation ratio in cases where the peak abundance ratio of the carbonyl-derived component in the total component of the C1s peak as measured by X-ray photoelectron spectroscopy (XPS) on the surface is not less than 1.0 atomic percent. It is also clear that use of a betaine compound, which has been thought to have no influence on the antithrombin activation capacity, in combination leads to decreases in not only the adhesion rate, but also the thrombin activation ratio.

INDUSTRIAL APPLICABILITY

The antithrombogenic material of present invention can be used for medical equipments and medical instruments requiring maintenance of high antithrombogenicity for a long period, in the field of medicine.

## Claims

1. An antithrombogenic material comprising:

    a coating material containing: a skeletal structure composed of a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; a skeletal structure composed of 4-(aminomethyl)benzenecarboxyimidamide or benzene amidine; and a skeletal structure composed of methoxybenzenesulfonic acid amide; and
    a base material whose surface is coated with said coating material;

    wherein

    said coating material is covalently bound to said base material; and
    the peak abundance ratio of the carbonyl-derived component in the total component of the Cls peak as measured by X-ray photoelectron spectroscopy (XPS) on the surface is not less than 1.0 atomic percent.

2. The antithrombogenic material according to claim 1, wherein said skeletal structure composed a polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; said skeletal structure composed of 4-(aminomethyl)benzenecarboxyimidamide or benzene amidine; and said skeletal structure composed of methoxybenzenesulfonic acid amide; are the compound represented by the following General Formula (I):

    [Chemical Formula 1]

    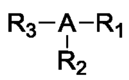 (I)

    [wherein $R_1$ represents said polymer containing, as a constituent monomer, a compound selected from the group consisting of ethylene glycol, propylene glycol, vinylpyrrolidone, vinyl alcohol, vinylcaprolactam, vinyl acetate, styrene, methyl methacrylate, hydroxyethyl methacrylate, and siloxane; $R_2$ represents 4-(aminomethyl)benzenecarboxyimidamide or benzene amidine; $R_3$ represents methoxybenzenesulfonic acid amide; and A represents an arbitrary covalent bond(s)].

3. The antithrombogenic material according to claim 1 or 2, wherein said coating material comprises at least one of a betaine compound, cationic polymer, anionic polymer, and anionic compound, and has an antithrombogenic action.

4. The antithrombogenic material according to claim 2 or 3, wherein

said coating material comprises a betaine compound; each of said compound represented by General Formula (I) and said betaine compound is covalently bound to said base material; and said coating material has an antithrombogenic action.

**5.** The antithrombogenic material according to claim 2 or 3, wherein said coating material comprises at least one of a cationic polymer, anionic polymer, and anionic compound; each of said compound represented by General Formula (I) and at least one of said cationic polymer, anionic polymer, and anionic compound is covalently bound to said base material; and said coating material has an antithrombogenic action.

**6.** The antithrombogenic material according to claim 2 or 3, wherein said coating material comprises at least one of a cationic polymer, anionic polymer, and anionic compound; at least one of said cationic polymer, anionic polymer, and anionic compound is covalently bound to said base material; said compound represented by General Formula (I) is covalently bound to any one of said cationic polymer, anionic polymer, and anionic compound; and said coating material has an antithrombogenic action.

**7.** The antithrombogenic material according to claim 2 or 3, wherein said coating material comprises a betaine compound, and at least one of a cationic polymer, anionic polymer, and anionic compound; at least one of said cationic polymer, anionic polymer, and anionic compound is covalently bound to said base material; each of said compound represented by General Formula (I) and said betaine compound is covalently bound to any one of said cationic polymer, anionic polymer, and anionic compound; and said coating material has an antithrombogenic action.

**8.** The antithrombogenic material according to any one of claims 2 to 7, wherein said compound represented by General Formula (I) is a compound represented by any of the following General Formulae (II) to (V):

[Chemical Formula 2]

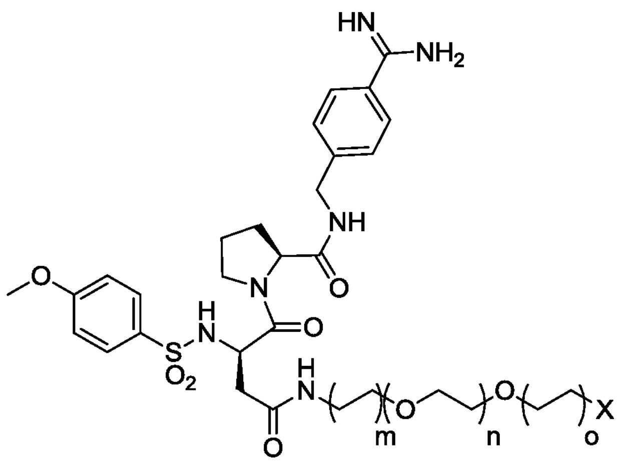

(II)

[Chemical Formula 3]

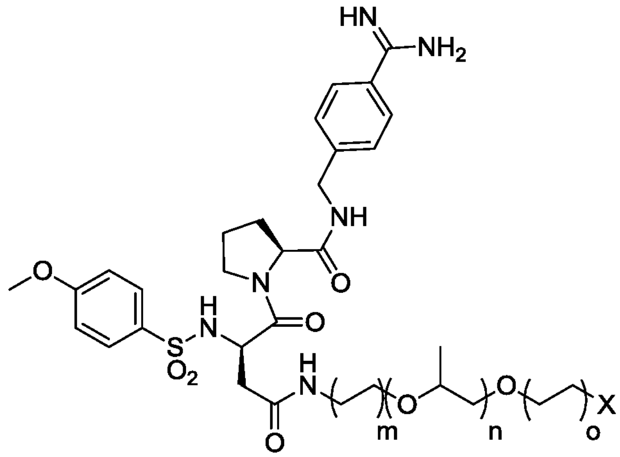

(III)

[Chemical Formula 4]

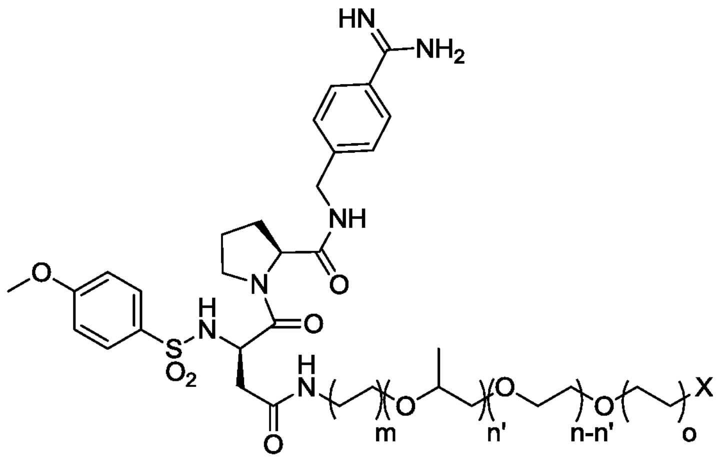

(IV)

[Chemical Formula 5]

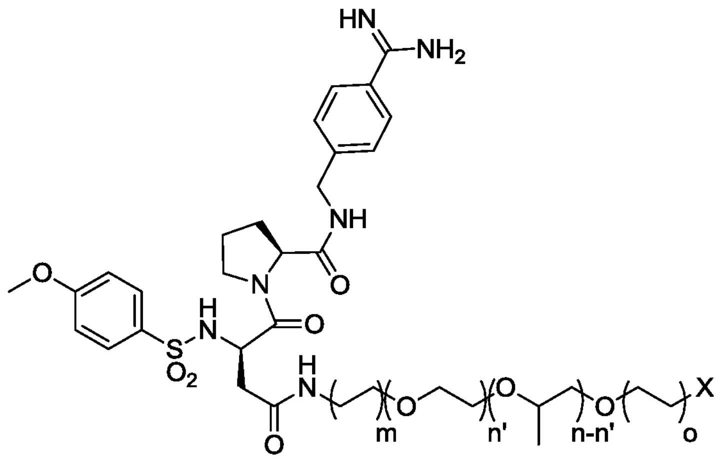

(V)

[wherein each of m and o represents an integer of 0 to 4; n represents an integer of 3 to 1000, and n' represents an integer of 3 to 1000, with the proviso that n≥n'; and X represents a functional group selected from the group consisting of hydroxyl, thiol, amino, carboxyl, aldehyde, isocyanate, and thioisocyanate].

**9.** The antithrombogenic material according to any one of claims 2 to 8, wherein

said cationic polymer is a polymer comprising, as a constituent monomer, a compound selected from the group consisting of alkyleneimines, vinylamines, allylamines, lysine, protamine, and diallyldimethylammonium chloride;
said anionic polymer is a polymer comprising, as a constituent monomer, a compound selected from the group consisting of acrylic acid, methacrylic acid, α-glutamic acid, γ-glutamic acid, and aspartic acid; and
said anionic compound is a compound selected from the group consisting of oxalic acid, malonic acid, succinic acid, fumaric acid, glutaric acid, adipic acid, pimelic acid, suberic acid, azelaic acid, sebacic acid, malic acid, tartaric acid, and citric acid.

**10.** The antithrombogenic material according to any one of claims 1 to 9, wherein said coating material has a thickness of 1 to 600 nm and is present on the surface of said base material.

**11.** The antithrombogenic material according to any one of claims 1 to 10, wherein said coating material is present to a depth of 15 to 200 nm from the interface of said base material.

**INTERNATIONAL SEARCH REPORT**

International application No.
PCT/JP2014/081306

A. CLASSIFICATION OF SUBJECT MATTER
*A61L33/00*(2006.01)i

According to International Patent Classification (IPC) or to both national classification and IPC

B. FIELDS SEARCHED

Minimum documentation searched (classification system followed by classification symbols)
A61L15/00-33/18

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY/MEDLINE/EMBASE/BIOSIS(STN),
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| P,X | WO 2014/168197 A1 (Toray Industries, Inc.), 16 October 2014 (16.10.2014), claims; examples (Family: none) | 1-11 |
| X<br>Y | JP 2009-225824 A (Toray Industries, Inc.), 08 October 2009 (08.10.2009), claims; paragraphs [0014], [0025] to [0028]; examples (Family: none) | 1-3,5,6,8-11<br>4,7 |
| X<br>Y | WO 2008/032758 A1 (Toray Industries, Inc.), 20 March 2008 (20.03.2008), claims; paragraphs [0016], [0027] to [0030]; examples & US 2010/0176048 A1 & EP 2072070 A1 | 1-3,5,6,8-11<br>4,7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 February 2015 (04.02.15) | 17 February 2015 (17.02.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

INTERNATIONAL SEARCH REPORT

International application No. PCT/JP2014/081306

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2013/038901 A1 (Nissan Chemical Industries, Ltd.), 21 March 2013 (21.03.2013), paragraph [0005] & TW 201329094 A1 | 4,7 |
| A | JP 2006-291193 A (Toray Industries, Inc.), 26 October 2006 (26.10.2006), examples (Family: none) | 1-11 |
| A | JP 2010-082067 A (Toray Industries, Inc.), 15 April 2010 (15.04.2010), claims (Family: none) | 1-11 |
| A | JP 2004-525888 A (Haemosys GmbH), 26 May 2004 (26.05.2004), claims; paragraph [0132] & US 2005/0070479 A1 claims; paragraph [0275] & EP 1355674 A2 & WO 2002/059065 A2 | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4152075 B
- JP 3497612 B
- JP 10513074 PCT
- JP 60041947 B
- JP 60047287 B
- JP 4273965 B
- JP 10151192 A
- JP 3341503 B
- JP 3834602 B
- JP 4461217 B
- WO 08032758 A
- WO 12176861 A
- JP 54063025 A
- JP 2000279512 A
- JP 5349873 B